# EUROPEAN PATENT APPLICATION

(11) **EP 3 620 172 A1**
(43) Date of publication of application: **11.03.2020**
(21) Application number: 19203794.3
(22) Date of filing: 11.10.2013
(51) Int. Cl.: A61K 39/00, A61K 39/102, A61K 39/29

(54) **NON-CROSS-LINKED ACELLULAR PERTUSSIS ANTIGENS FOR USE IN COMBINATION VACCINES**

(30) Priority: 12.10.2012 US 201261713356 P
(62) Divisional of application: 13774463.7
(71) Applicant: GlaxoSmithKline Biologicals SA, 1330 Rixensart (BE)
(72) Inventor: TARLI, Lorenzo, 53100 Siena (IT); CONTORNI, Mario, 53100 Siena (IT); BARTALESI, Alessandro, 53100 Siena (IT)
(74) Representative: Evans, Stephen John Eves

(57) **Abstract**

The present invention relates to stable compositions comprising acellular pertussis antigens that have not been cross-linked with a cross-linking agent such as formaldehyde or glutaraldehyde and their use as acellular pertussis components in combination vaccines. Processes for preparing these antigens and compositions are also disclosed.

## Description

This application claims the benefit of US provisional patent application 61/713,356 filed October 12th 2012, the complete contents of which are incorporated herein by reference for all purposes.

### FIELD OF THE INVENTION

The present invention relates to stable compositions comprising acellular pertussis antigens that have not been cross-linked with a cross-linking agent such as formaldehyde or glutaraldehyde and their use as acellular pertussis components in combination vaccines.

### BACKGROUND

*Bordetella pertussis* causes whooping cough. Pertussis antigens in vaccines are either cellular (whole cell, in the form of inactivated *B. pertussis* cells; 'wP') or acellular ('aP'). Preparation of cellular pertussis antigens is well documented (e.g. see chapter 21 of reference 1) *e.g.* it may be obtained by heat inactivation of a phase I culture of *B. pertussis.* Acellular pertussis antigens comprise detoxified pertussis toxin (pertussis toxoid, or 'PT'); (2) filamentous hemagglutinin ('FHA'); and (3) pertactin (also known as the '69 kiloDalton outer membrane protein'). These three antigens are typically prepared by isolation from a *B. pertussis* culture grown in modified Stainer-Scholte liquid medium. *B. pertussis* fimbriae (*e.g.* agglutinogens 2 and 3) are additionally included in some pertussis vaccines.

PT and FHA can be isolated from the fermentation broth (*e.g.* by adsorption on hydroxyapatite gel), whereas pertactin can be extracted from the cells by heat treatment and flocculation (*e.g*. using barium chloride). The antigens are then purified in successive chromatographic and/or precipitation steps. PT and FHA can be purified by hydrophobic chromatography, affinity chromatography and size exclusion chromatography. Pertactin can be purified by ion exchange chromatography, hydrophobic chromatography and size exclusion chromatography.

FHA and pertactin are typically treated with formaldehyde as cross-linking agent prior to their inclusion in a vaccine formulation. PT is typically detoxified by treatment with cross-linking agents such as formaldehyde or glutaraldehyde. As an alternative to this chemical detoxification procedure, PT can be genetically detoxified by the substitution of amino acids that are required for the enzymatic activity and therefore responsible for the toxicity of the antigen [2]. However, genetically detoxified PT can be unstable and prone to degradation when stored in liquid form [3] and so treatment with low concentrations of formaldehyde has been used to stabilise the protein without affecting its physicochemical and immunological parameters [4].

Cross-linking of an antigen with a cross-linking agent such as formaldehyde or glutaraldehyde is an additional processing step that adds to the costs associated with preparing a vaccine and introduces an additional variable in the overall production process. However, safety concerns, in particular when the FHA and pertactin are isolated from the same *B. pertussis* culture as enzymatically active PT, have led to the adoption of manufacturing processes in which all *B. pertussis* antigen components are inactivated, preferably by formaldehyde treatment, prior to inclusion in a vaccine. In addition, formaldehyde treatment is considered to be beneficial in increasing the stability of each of the individual *B. pertussis* antigens in liquid vaccine formulations.

It has now been found that extensive purification of genetically detoxified PT and other *B. pertussis* antigens from a *B. pertussis* strain carrying the gene encoding genetically detoxified PT makes chemical cross-linking of the PT and other *B. pertussis* antigens unnecessary. The individually purified components retain the stability and immunogenicity even after prolonged storage at elevated temperatures (at least one month at 37°C) without the necessity for treatment with formaldehyde or other cross-linking agents.

### SUMMARY OF THE INVENTION

The present invention provides safe and immunogenic vaccine compositions that no longer require treatment of the *B. pertussis* antigens with a cross-linking agent. The vaccine formulations of the invention can be stored in the same way as standard combination vaccine comprising acellular pertussis components without losing potency due to degradation of the antigens during storage. In particular, the invention relates to vaccine compositions comprising at least two non-cross-linked *B. pertussis* antigens selected from PT, FHA and pertactin with the proviso that any PT is genetically detoxified.

The invention also relates to a combination vaccine comprising a genetically detoxified pertussis toxin and one or more additional antigen selected from IPV, HBsAg and Hib, characterised in that the genetically detoxified pertussis toxin is not treated with a cross-linking agent.

In another embodiment, the invention relates to a combination vaccine comprising *B. pertussis* antigens PT, FHA and pertactin, characterised in that at least two of the *B. pertussis* antigens are not treated with a cross-linking agent, with the proviso that, if PT is not treated with a cross-linking agent, PT is a genetically detoxified pertussis toxin.

In a further embodiment, the invention relates to a combination vaccine comprising an aluminium salt adjuvant and at least two non-cross-linked *B. pertussis* antigens selected from PT, FHA and pertactin with the proviso that any PT is genetically detoxified. The concentration of Al⁺⁺⁺ is preferably less than 1 mg/ml. The combination vaccine of the invention may further include one or more Toll-like receptor (TLR) agonist, which may be adsorbed to an aluminium salt adjuvant. For example, the combination vaccine of the invention may include a TLR4 agonist (*e.g.* 3d-MPL) or a TLR7 agonist (*e.g.* compound T, see below) which is typically adsorbed to an aluminium salt adjuvant.

In yet a further aspect, the invention relates to a combination vaccine comprising an oil-in-water emulsion adjuvant and at least two non-cross-linked *B. pertussis* antigens selected from PT, FHA and pertactin with the proviso that any PT is genetically detoxified. Oil-in-water emulsion adjuvants used in the combination vaccine of the invention include MF59 and/or AS03.

In one embodiment, the invention relates to a preservative-free combination vaccine comprising at least two non-cross-linked *B. pertussis* antigens selected from PT, FHA and pertactin with the proviso that any non-cross-linked PT is genetically detoxified.

In another embodiment, the invention relates to a combination vaccine comprising at least two non-cross-linked *B. pertussis* antigens selected from PT, FHA and pertactin with the proviso that any PT is genetically detoxified, wherein the weight ratio of PT:FHA:pertactin is 1:1:2 or 16:16:5 or 5:10:6 or 20:20:3 or 25:25:8 or 10:5:3.

In a further embodiment, the invention relates to a combination vaccine comprising D, T, aP, wherein the ratio of D to T measured in Lf units is between 2:1 and 3:1 and the aP component comprises at least two non-cross-linked *B. pertussis* antigens selected from PT, FHA and pertactin with the proviso that any PT is genetically detoxified.

In another embodiment, the invention relates to a combination vaccine comprising D, T, aP, wherein the ratio of T to D measured in Lf units is greater than 1.5 and the aP component comprises at least two non-cross-linked *B. pertussis* antigens selected from PT, FHA and pertactin with the proviso that any PT is genetically detoxified.

In a preferred embodiment, the combination vaccine of the invention includes a genetically detoxified pertussis toxin such as PT-9K/129G.

The invention further relates to a process for preparing an aP component comprising growing a culture of a *B. pertussis* strain expressing a genetically detoxified PT, purifying two or more *B. pertussis* antigens from the culture to obtain two or more batches each containing a different purified *B. pertussis* antigen, and mixing the two or more batches to prepare the aP component, wherein the process is characterised in that the purified *B. pertussis* antigens are not treated with a cross-linking agent.

In another embodiment, the invention relates to a process for preparing an aP component comprising growing a culture of a *B. pertussis* strain in which the gene encoding PT has been deleted, purifying two or more *B. pertussis* antigens from the culture to obtain two or more batches each containing a different purified *B. pertussis* antigen, and mixing the two or more batches to prepare the aP component, wherein the process is characterised in that the purified *B. pertussis* antigens are not treated with a cross-linking agent.

### DETAILED DESCRIPTION OF THE INVENTION

### Pertussis Toxin

Pertussis toxin is a protein of 105 kilodaltons composed of five subunits, named S1 to S5. Only the S1 subunit has enzymatic activity and confers a toxic effect on eukaryotic cells. Subunits S2, S3, S4, and S5 are present in a 1:1:2:1 ratio and form a nontoxic oligomer (B oligomer) which binds receptors on the surface of eukaryotic cells and delivers the toxic subunit S1 across the eukaryotic cell membrane. By replacing the codons of amino acids essential for the enzymatic activity of the S1 subunit in the chromosome of a *B. pertussis* strain, a *B. pertussis* strain that produces a genetically detoxified PT can be obtained.

Two regions of pertussis toxin which share sequence homology with the amino acid sequence of cholera toxin (CT) and *E. coli* heat-labile toxin (LT) and contribute to the ADP-ribosyltransferase activity of PT were identified by substitution mutagenesis. The first region is located between residues 8 and 13. In particular, mutant S1 in which Arg-9 is substituted with Lys-9 was found to have greatly reduced enzymatic activity [5]. A second region of S1, located between residues 51 and 59 was also shown to be involved in the toxicity of pertussis toxin, e.g. when Arg-58 was changed to Glu-58 [6]. The glutamic acid residue at position 129 in the S1 subunit was found to be part of the enzymatic site by photocross-linking experiments using radiolabeled NAD [7]. Substitution of residue 129 yields a PT mutant with substantially reduced enzymatic activity and hence very low toxicity. Pertussis toxin has also been detoxified by mutating Trp-26, His-35 and Cys-41 [6].

Single mutations of S1 result in up to a 1,000-fold reduction in pertussis toxin's toxic and enzymatic activities, while mutants with multiple substitutions in the S1 subunit (Lys-9 Gly-129, Glu-58 Gly-129, and Lys-9 Glu-58 Gly-129) are about 10⁶-fold detoxified [6].

In accordance with the present invention, preferred mutant forms of pertussis toxin have reduced or undetectable toxicity but retain the ability to induce protective immunity. These include S1 double mutants PT-9K/129G, PT-13L/129G, and PT-26I/129G. Preferably, the genetically detoxified PT is PT-9K/129G, *i.e.* with lysine at residue 9 and glycine at residue 129 as described in reference 8.

### Vaccine Compositions

The invention relates to vaccine compositions comprising at least two *B. pertussis* antigens selected from PT, FHA and pertactin that have not been treated with a cross-linking reagent, with the proviso that any PT is genetically detoxified. Extensive cross-linking of a protein antigen with a cross-linking agent such as formaldehyde or glutaraldehyde can interfere with the cleavage of the antigen by antigen presenting cells or the structural flexibility normally associated with an antigen when present in its native state. For example, formaldehyde treatment leads to the modification of the amino acids (mainly lysine) in the *B. pertussis* antigens and therefore induces structural and functional modification. Non-cross-linked *B. pertussis* antigens can be distinguished from cross-linked *B. pertussis* antigens by trypsin digestion since cross-linked antigens display different protease sensitivity from non-cross-linked antigens.

The non-cross-linked *B. pertussis* antigens of the present invention may display higher potency than conventional, cross-linked *B. pertussis* antigens. Therefore, in comparison to conventional vaccines in which all *B. pertussis* antigens have been crosslinked, smaller amounts of each of the non-cross-linked *B. pertussis* antigens may be needed to yield similar levels of protective immunity. In addition or alternatively, when the non-cross-linked *B. pertussis* antigens are administered in the same dose as conventional, cross-linked *B. pertussis* antigens, immunogenicity may be enhanced in comparison to the levels of immunogenicity achieved with the conventional, cross-linked *B. pertussis* antigens.

In a preferred embodiment of the invention, one of the *B. pertussis* antigens is a genetically detoxified pertussis toxin (*e.g*. PT-9K/129G). Vaccine compositions comprising genetically detoxified pertussis toxin typically require smaller amounts of PT to achieve comparable or greater immunogenicity than PT detoxified by conventional cross-linking with formaldehyde or glutaraldehyde, as genetically detoxified PT has been found to be more immunogenic than PT produced by conventional detoxification [9, 10]. Immunogenicity may be further enhanced because the non-cross-linked, genetically detoxified PT is more effectively processed by antigen presenting cells and provides a better substrate for a wider range of T-cell receptors due to its greater structural flexibility [11]. Thus, the amount of genetically detoxified PT in the vaccine compositions of the invention, in which the cross-linking step has been omitted, may be further reduced without affecting the immunogenicity of the vaccine compositions. Alternatively, the same amount of genetically detoxified PT may be used in place of a conventional, cross-linked PT component in a vaccine composition to achieve greater immunogenicity against the PT antigen.

In a further preferred embodiment of the invention, all three *B. pertussis* antigens PT, FHA and pertactin have not been treated with a cross-linking agent before inclusion in a vaccine composition. In such an embodiment, detoxification of PT is achieved genetically using a *B. pertussis* strain in which the gene encoding the S1 subunit of pertussis toxin has been mutated such that the mutant form lacks all or substantially all enzymatic activity.

However, compositions in which only FHA and pertactin have not been subjected to treatment with a cross-linking agent may also be useful as any reduction in the use of a cross-linking agent simplifies production and reduces the production of chemical waste. In addition, the overall content of residual formaldehyde present in the final vaccine formulation may sufficiently be reduced to be non-detectable, therefore reducing the likelihood of a negative response to formaldehyde in patients particularly prone to such a reaction.

The present invention therefore relates to an acellular pertussis (aP) component comprising at least two non-cross-linked *B. pertussis* antigens selected from PT, FHA and pertactin, with the proviso that any PT is genetically detoxified. Preferably, the aP component of the invention comprises or consists of non-cross-linked FHA, non-cross-linked pertactin and non-cross-linked, genetically detoxified PT. Alternatively, only FHA and pertactin have not been cross-linked with a cross-linking agent such as formaldehyde or glutaraldehyde.

Quantities of each of the antigens in the aP component are typically expressed in µg. Vaccines of the invention typically include between 2-30 µg PT per unit dose. In a first type of vaccine, PT can be present at between 5-30µg per unit dose (*e.g.* 5, 7.5, 20 or 25µg), whereas in a second type vaccine the composition will generally include between 2-10µg PT per unit dose (*e.g.* 2.5µg or 8µg). Where a vaccine includes FHA, it is typically present between 2-30 µg per unit dose. In a first type of vaccine, FHA can be present at between 2.5-25µg per unit dose (*e.g.* 2.5, 5, 10, 20 or 25µg), whereas in a second type FHA can be present at between 4-10µg per unit dose (*e.g.* 5µg or 8µg). Where a vaccine includes pertactin, this is typically present between 2-10 µg per unit dose. In a first type of vaccine, pertactin can be present at between 2.5-10µg per unit dose (*e.g.* 2.5, 3, 8 or 10µg), whereas in a second type vaccine pertactin can be present at between 2-3µg per unit dose (*e.g.* 2.5µg or 3µg).

Thus a booster vaccine for adolescents and adults typically contains 2.5 to 8 µg PT, between 4 and 8 µg FHA and between 2.5 and 8 µg pertactin per unit dose. Preferably, a booster vaccine comprises 4 µg PT, 4 µg FHA and 8 µg pertactin, more preferably 5 µg PT, 2.5 µg FHA and 2.5 µg pertactin per unit dose. A paediatric vaccine preferably comprises 7 µg or 7.5 µg PT, 10 µg FHA and 10 µg pertactin, per unit dose. The unit dose volume usually administered is 0.5 ml.

A vaccine normally contains ≤80µg per unit dose of total acellular pertussis antigens. Each individual antigen will usually be present at ≤30µg per unit dose.

It is usual that each of PT, FHA and pertactin are present in a vaccine of the invention. These may be present at various amounts, such as PT:FHA:pertactin amounts (µg) of 20:20:3, 25:25:8, 16:16:5, 5:10:6, 4:4:8, 5:2.5:2.5, 7.5:10:10, or 10:5:3. Multiples of these amounts can also be used *e.g.* 10:10:1.5, or 30:30:4.5, or 20:10:6, *etc.* One useful vaccine includes 4µg PT, 4µg FHA and 8µg pertactin. It is usual to have a mass excess of FHA relative to pertactin if both are present.

If a vaccine includes an aluminium salt adjuvant then PT in the vaccine is preferably adsorbed (sometimes totally adsorbed) onto an aluminium salt, preferably onto an aluminium hydroxide adjuvant. Any FHA can also be adsorbed to the aluminium salt. Any pertactin can be adsorbed to the aluminium salt adjuvant, but the presence of pertactin normally means that the composition requires the presence of aluminium hydroxide to ensure stable adsorption [12].

For acellular pertussis, a modified intracerebral mouse protection assay (MICA) is used to determine potency. MICA is a lethal challenge model in mice which detects mouse protective activity provided by an acellular pertussis vaccine. The potency of each final bulk is expressed as a relative potency to a reference vaccine. That reference vaccine is calibrated against the International Standard for acellular pertussis vaccines (currently JINH-3) and the protective activity is expressed in International Units. The potency of the vaccine should be at least 4.0 IU in the volume recommended for a single human dose, *i.e.* at least 8 IU/ml.

### Manufacturing of aP Components

The present invention further relates to manufacturing processes for preparing non-cross-linked *B. pertussis* antigens that can be used for preparing aP components present in the vaccine compositions of the invention. *B. pertussis* antigens that may be purified from a *B. pertussis* culture and included in the aP component according to the invention are PT, FHA, pertactin, agglutinogen 2 and agglutinogen 3. Preferably, the aP component includes PT, FHA and (optionally) pertactin. The purified antigens forming the aP component are usually mixed in specific ratios that have been found to be particularly suitable in raising a protective antibody response against all *B. pertussis* antigens included in the aP component. For example, PT, FHA and pertactin may be mixed in the following weight ratios of PT:FHA:pertactin: 1:1:2 or 16:16:5 or 5:10:6 or 20:20:3 or 25:25:8 or 10:5:3.

In one aspect, the invention relates to a process for preparing an aP component comprising growing a culture of a *B. pertussis* strain expressing a genetically detoxified PT, purifying two or more *B. pertussis* antigens from the culture to obtain two or more batches each containing a different purified *B. pertussis* antigen, and mixing the two or more batches in the desired ratios (see above) to prepare the aP component, wherein the process is characterised in that the purified *B. pertussis* antigens are not treated with a cross-linking agent (*i.e.* the antigens remain in their non-crosslinked native form in the final vaccine). Using a *B. pertussis* strain encoding genetically detoxified PT has the advantage that any carry-over of toxin activity from the fermentation medium to components comprising another *B. pertussis* antigen can be avoided and therefore a precautionary treatment of the *B. pertussis* antigens other than PT for safety reasons becomes unnecessary. The genetically detoxified pertussis toxin encoded by the *B. pertussis* strain used for preparing the culture is preferably PT-9K/129G.

In another aspect, the invention relates to a process for preparing an aP component comprising growing a culture of a *B. pertussis* strain in which the gene encoding PT has been deleted, purifying two or more *B. pertussis* antigens from the culture to obtain two or more batches each containing a different purified *B. pertussis* antigen, and mixing the two or more batches in the desired ratios to prepare the aP component, wherein the process is characterised in that the purified *B. pertussis* antigens are not treated with a cross-linking agent. Using a mutant *B. pertussis* strain lacking the PT gene has the same advantage associated with using a *B. pertussis* strain encoding genetically detoxified PT, namely contamination of other *B. pertussis* antigens with toxin activity and the resulting need for a precautionary treatment of the *B. pertussis* antigens with a cross-linking agent are avoided.

In a third, less preferred aspect, the invention relates to a process for preparing an aP component comprising growing a culture of a *B. pertussis* strain, purifying two or more *B. pertussis* antigens from the culture to obtain two or more batches each containing a different purified *B. pertussis* antigen; and mixing the two or more batches in the desired ratios to prepare the aP component, wherein the process is characterised in that only the batch containing purified enzymatically active PT is treated with a cross-linking agent, but the batches containing other purified *B. pertussis* antigens are not treated with a cross-linking agent. Purification of *B. pertussis* antigens other than enzymatically active PT from a *B. pertussis* culture can be performed without the need for cross-linking of these *B. pertussis* antigens. However, additional safety testing may be necessary to ensure that no toxin activity has been carried over from the culture to the batches containing *B. pertussis* antigens other than enzymatically active PT.

By removing any cross-linking steps in the production process, the process can be shortened substantially and becomes more energy-efficient. For example, detoxification of pertussis toxin with formaldehyde can require an incubation period of 24-32 days at 37°C (see reference 13). As a consequence of the shortened production process, the production of multivalent vaccines can be optimised by producing D and T components that require detoxification first, followed by the production of the *B. pertussis* antigens and other components that do not require detoxification. This allows for the optimal use of fermentation and purification facilities and makes vaccine production commercially viable even in smaller-scale facilities. For example, the amount of space otherwise needed for incubating *B. pertussis* antigens with a cross-linking agent is no longer required. In addition, the environmental impact of the production process is reduced because less or no chemical waste in form of aqueous solutions containing residual cross-linking agent is produced during the manufacturing of the aP component and energy consumption is reduced due to the omission of a lengthy incubation period at 37°C for the *B. pertussis* antigens.

In general, the invention omits any cross-linking steps in the production process, and so the immunogens are referred to as being "not treated with a cross-linking agent", as "non-cross-linked", *etc.* In some embodiments, however, the invention can use a cross-linking agent but avoids the use of an aldehyde cross-linking agent. For instance, the invention can use a cross-linking agent but avoid the use of formaldehyde and glutaraldehyde. Preferably, though, no cross-linking agents are used for treating the *B.pertussis* immunogens.

### Combination Vaccines

The vaccine compositions of the invention will generally be combination vaccines *i.e.* including protective antigen(s) from at least one pathogen other than *B. pertussis.* The additional protective antigen(s) can be viral and/or bacterial. Typical bacterial pathogens include, but are not limited to: *Corynebacterium diphtheriae; Clostridium tetani*; *Haemophilus influenzae* type b; *Neisseria meningitidis*, including serogroups A, B, C, W135 and/or Y; and *Streptococcus pneumoniae*, including serotypes 6B, 14, 19F, and 23F. Typical viral pathogens include, but are not limited to: poliovirus; hepatitis A virus; hepatitis B virus; measles virus; mumps virus; rubella virus; and varicella zoster virus.

### Diphtheria

*Corynebacterium diphtheriae* causes diphtheria. Diphtheria toxin can be treated (*e.g.* using formalin or formaldehyde) to remove toxicity while retaining the ability to induce specific anti-toxin antibodies after injection. These diphtheria toxoids are used in diphtheria vaccines, and are disclosed in more detail in chapter 13 of reference 1. Preferred diphtheria toxoids are those prepared by formaldehyde treatment. The diphtheria toxoid can be obtained by growing *C.diphtheriae* in growth medium (*e.g.* Fenton medium, or Linggoud & Fenton medium), which may be supplemented with bovine extract, followed by formaldehyde treatment, ultrafiltration and precipitation. Preferably the growth medium for growing *C.diphtheriae* is free from animal-derived components. The toxoided material may then be treated by a process comprising sterile filtration and/or dialysis. Alternatively, genetically detoxified diphtheria toxin (*e.g*., CRM197) may be used which typically requires formaldehyde-treatment to maintain long-term stability during storage.

The diphtheria toxoid is preferably adsorbed onto an aluminium hydroxide adjuvant.

Quantities of diphtheria toxin and/or toxoid in a composition are generally measured in the 'Lf unit ("flocculating units", or the "limes flocculating dose", or the "limit of flocculation"), defined as the amount of toxin/toxoid which, when mixed with one International Unit of antitoxin, produces an optimally flocculating mixture [14,15]. For example, the NIBSC supplies 'Diphtheria Toxoid, Plain' [16], which contains 300 LF per ampoule, and also supplies 'The 1st International Reference Reagent For Diphtheria Toxoid For Flocculation Test' [17] which contains 900 Lf per ampoule. The concentration of diphtheria toxin or toxoid in a composition can readily be determined using a flocculation assay by comparison with a reference material calibrated against such reference reagents.

The immunizing potency of diphtheria toxoid in a composition is generally expressed in international units (IU). The potency can be assessed by comparing the protection afforded by a composition in laboratory animals (typically guinea pigs) with a reference vaccine that has been calibrated in IUs. NIBSC supplies the 'Diphtheria Toxoid Adsorbed Third International Standard 1999' [18,19], which contains 160 IU per ampoule, and is suitable for calibrating such assays.

A three-dilution assay can be used to determine the potency of the compositions of the invention. After immunization, guinea-pigs are bled or challenged either by the subcutaneous or by the intradermal route. In an alternative embodiment, mice are used in place of guinea pigs. When guinea pigs or mice are bled, the antitoxin levels of the individual animals are titrated by means of toxin neutralization tests performed using *in vivo* or *in vitro* serological methods that have been validated on vaccines of the types being tested. In one embodiment, diphtheria toxoids produced in fermentation medium comprising animal-derived components are used for validation. The potency of the composition of the invention is calculated using appropriate statistical methods. For three-dilution assays, the limits of the 95% confidence intervals of the estimate of potency is within 50-200% of the estimated potency unless the lower limit of the 95% confidence interval of the estimated potency is greater than 30 IU per single human dose. When one-dilution tests are performed, the potency of the test vaccine is demonstrated to be significantly greater than 30 IU per human dose.

By IU measurements, compositions generally include at least 30 IU/dose. Compositions typically include between 20 and 80 Lf/ml of diphtheria toxoid, typically about 50 Lf/ml. Booster vaccines for adolescents and adults typically contain between 4 Lf/ml and 8 Lf/ml of diphtheria toxoid, *e.g*., 2.5 Lf, preferably 4 Lf, per 0.5 ml dose. Paediatric vaccines typically contain between 20 and 50 Lf/ml of diphtheria toxoid, e.g. 10 Lf or 25 Lf per 0.5 ml dose.

Purity of a protein preparation can be expressed by the ratio of specific protein to total protein. The purity of diphtheria toxoid in a composition is generally expressed in units of Lf diphtheria toxoid per unit mass of protein (nondialysable) nitrogen. For instance, a very pure toxin/toxoid might have a purity of more than 1700 Lf/mg N, indicating that most or all of the protein in the composition is diphtheria toxin/toxoid [20].

### Tetanus

*Clostridium tetani* causes tetanus. Tetanus toxin can be treated to give a protective toxoid. The toxoids are used in tetanus vaccines, and are disclosed in more detail in chapter 27 of reference 1. Thus a combination vaccine of the invention can include a tetanus toxoid. Preferred tetanus toxoids are those prepared by formaldehyde treatment. The tetanus toxoid can be obtained by growing *C.tetani* in growth medium (*e.g.* a Latham medium derived from bovine casein), followed by formaldehyde treatment, ultrafiltration and precipitation Preferably the growth medium for growing *C*. *tetani* is free from animal-derived components. The material may then be treated by a process comprising sterile filtration and/or dialysis.

The tetanus toxoid is preferably adsorbed onto an aluminium hydroxide adjuvant.

Quantities of tetanus toxoid can be expressed in 'Lf' units (see below), defined as the amount of toxoid which, when mixed with one International Unit of antitoxin, produces an optimally flocculating mixture [14]. The NIBSC supplies 'The 1st International Reference Reagent for Tetanus Toxoid For Flocculation Test' [21] which contains 1000 LF per ampoule, by which measurements can be calibrated.

Booster vaccines for adolescents and adults typically contain 5 Lf of tetanus toxoid per 0.5 ml dose. Paediatric vaccines typically contain between 5 and 10 Lf of tetanus toxoid per 0.5 ml dose.

The immunizing potency of tetanus toxoid is measured in international units (IU), assessed by comparing the protection afforded by a composition in laboratory animals (typically guinea pigs) with a reference vaccine e.g. using NIBSC's 'Tetanus Toxoid Adsorbed Third International Standard 2000' [22,23], which contains 469 IU per ampoule. The potency of tetanus toxoid in a composition of the invention should be at least 35 IU per dose *e.g.* at least 70 IU/ml. More preferably, the potency of tetanus toxoid in a composition of the invention is at least 40 IU per dose. However, in booster vaccines for adults and adolescents, a reduced potency of 20 IU/dose may be acceptable because of the reduced antigen content in comparison to paediatric vaccine intended for primary immunization.

A multiple dilution assay can be used to determine the potency of the compositions of the invention. After immunization, guinea-pigs are bled or challenged either by the subcutaneous or by the intradermal route. In an alternative embodiment, mice are used in place of guinea pigs. When guinea pigs or mice are bled, the antitoxin levels of the individual animals are titrated by means of toxin neutralization tests performed using *in vivo* or *in vitro* serological methods that have been validated on vaccines of the types being tested. The potency of the composition of the invention is calculated using appropriate statistical methods. Where multiple dilution assays are used, the lower and upper limits of 95% confidence interval should be within 50-200% of the estimated potency respectively. The lower 95% confidence limit of the estimated potency of a tetanus vaccine used for the primary immunization of children should not be less than 40 IU per single human dose.

The purity of tetanus toxoid in a composition is generally expressed in units of Lf tetanus toxoid per unit mass of protein (nondialysable) nitrogen. The tetanus toxoid should have a puity of at least 1000 Lf/mg N. *Hib*

*Haemophilus influenzae* type b ('Hib') causes bacterial meningitis. Hib vaccines are typically based on the capsular saccharide antigen (*e.g*. chapter 14 of reference 1), the preparation of which is well documented (*e.g.* references 24 to 33). The *H.influenzae* bacteria can be cultured in the absence of animal-derived components. The Hib saccharide is conjugated to a carrier protein in order to enhance its immunogenicity, especially in children. Typical carrier proteins in these conjugates are tetanus toxoid, diphtheria toxoid, the CRM197 derivative of diphtheria toxin, or an outer membrane protein complex from serogroup B meningococcus. Thus a combination vaccine of the invention can include a Hib capsular saccharide conjugated to a carrier protein.

Any suitable activation chemistry and/or linker chemistry can be used in the conjugation of Hib saccharides. The saccharide will typically be activated or functionalised prior to conjugation. Activation may involve, for example, cyanylating reagents such as CDAP (*e.g*. 1-cyano-4-dimethylamino pyridinium tetrafluoroborate [34, 35]). Other suitable techniques use carbodiimides, hydrazides, active esters, norborane, p-nitrobenzoic acid, N-hydroxysuccinimide, S-NHS, EDC, TSTU; see also the introduction to reference 36).

Linkages via a linker group may be made using any known procedure, for example, the procedures described in references 37 and 38. One type of linkage involves reductive amination of the polysaccharide, coupling the resulting amino group with one end of an adipic acid linker group, and then coupling a protein to the other end of the adipic acid linker group [39, 40, 41]. Other linkers include B-propionamido [42], nitrophenyl-ethylamine [43], haloacyl halides [44], glycosidic linkages [45, 46, 47], 6-aminocaproic acid [48], ADH [49], C₄ to C₁₂ moieties [50] *etc.* As an alternative to using a linker, direct linkage can be used. Direct linkages to the protein may comprise oxidation of the polysaccharide followed by reductive amination with the protein, as described in, for example, references 46 and 51.

Tetanus toxoid is a preferred carrier, as used in the product commonly referred to as 'PRP-T'. PRP-T can be made by activating a Hib capsular polysaccharide using cyanogen bromide, coupling the activated saccharide to an adipic acid linker (such as (1-ethyl-3-(3-dimethylaminopropyl) carbodiimide), typically the hydrochloride salt), and then reacting the linker-saccharide entity with a tetanus toxoid carrier protein.

The CRM197 diphtheria toxoid is another preferred Hib carrier protein [52,53,54]. A preferred conjugate comprises the Hib saccharide covalently linked to CRM197 via adipic acid succinic diester [55,56].

The saccharide moiety of the conjugate may comprise full-length polyribosylribitol phosphate (PRP) as prepared from Hib bacteria, and/or fragments of full-length PRP. Conjugates with a saccharide:protein ratio (w/w) of between 1:5 (*i.e.* excess protein) and 5:1 (*i.e.* excess saccharide) may be used *e.g.* ratios between 1:2 and 5:1 and ratios between 1:1.25 and 1:2.5. In preferred vaccines, however, the weight ratio of saccharide to carrier protein is between 1:2.5 and 1:3.5. In vaccines where tetanus toxoid is present both as an antigen and as a carrier protein then the weight ratio of saccharide to carrier protein in the conjugate may be between 1:0.3 and 1:2 [57].

Quantities of Hib antigens are typically expressed in µg of saccharide. The concentration of saccharide in a vaccine is typically between 10-30µg/ml *e.g.* 20µg/ml. Administration of the Hib conjugate preferably results in an anti-PRP antibody concentration of ≥0.15µg/ml, and more preferably ≥1µg/ml, and these are the standard response thresholds.

### Meningococcus

*Neisseria meningitidis* causes bacterial meningitis. Based on the organism's capsular polysaccharide, various serogroups of *N.meningitidis* have been identified, including A, B, C, H, I, K, L, 29E, W135, X, Y & Z. The serogroups most associated with disease are A, B, C, W135 and Y. Current vaccines against serogroups A, C, W135 and Y are based on the capsular saccharide antigens, but this approach is not suitable for serogroup B, and so protein antigens and outer-membrane vesicles are used instead [58]. The capsular saccharides are conjugated to carrier proteins in order to enhance immunogenicity. Typical carrier proteins are tetanus toxoid (as in the NIMENRIX™ product), diphtheria toxoid (as in the MENACTRA™ product), and the CRM197 derivative of diphtheria toxin (as in the MENVEO™ product). Thus a combination vaccine of the invention can include one or more (*e.g.* 2, 3, or 4) of capsular saccharides, conjugated to a carrier protein, selected from: (1) serogroup A *N.meningitidis*; (2) serogroup C *N.meningitidis*; (3) serogroup W135 *N.meningitidis*; and/or (4) serogroup Y *N.meningitidis.* Saccharides are individually conjugated to the same or different carrier proteins (*e.g*. all to CRM197 or tetanus toxoid) and subsequently mixed to obtain a combination vaccine including more than one capsular saccharide.

The saccharide moiety of the conjugate may comprise full-length saccharide as prepared from meningococci, and/or fragments thereof. Serogroup C saccharides may be prepared from either OAc+ or OAc- strains. For serogroup A saccharides, preferably at least 50% (*e.g.* at least 60%, 70%, 80%, 90%, 95% or more) of the mannosamine residues are O-acetylated at the C-3 position. Meningococcal conjugates with a saccharide:protein ratio (w/w) of between 1:10 (*i.e.* excess protein) and 10:1 (*i.e.* excess saccharide) may be used *e.g.* ratios between 1:5 and 5:1, between 1:2.5 and 2.5:1, or between 1:1.25 and 1.25:1.

The *N.meningitidis* bacteria can be cultured in the absence of animal-derived components.

Quantities of meningococcal antigens are typically expressed in µg of saccharide. The concentration of saccharide in a vaccine is typically between 5-30µg/ml per serogroup *e.g.* 10µg/ml or 20µg/ml. Administration of a conjugate preferably results in an increase in serum bactericidal assay (SBA) titre for the relevant serogroup of at least 4-fold, and preferably at least 8-fold. SBA titres can be measured using baby rabbit complement or human complement [59].

### Pneumococcus

*Streptococcus pneumoniae* causes bacterial meningitis. Like Hib and meningococcus, existing vaccines are based on capsular saccharides. The *S.pneumoniae* bacteria can be cultured in the absence of animal-derived components. Thus a combination vaccine of the invention can include a pneumococcal capsular saccharide conjugated to a carrier protein.

It is preferred to include saccharides from more than one serotype of *S.pneumoniae,* and particularly at least serotypes 6B, 14, 19F and 23F. Further serotypes are preferably selected from: 1, 3, 4, 5, 7F, 9V and 18C. For example, mixtures of polysaccharides from 23 different serotype are widely used, as are conjugate vaccines with polysaccharides from between 5 and 11 different serotypes [60]. For example, PREVNAR™ [61] contains conjugated saccharides from seven serotypes (4, 6B, 9V, 14, 18C, 19F, and 23F), SYNFLORIX™ contains conjugated saccharides from ten serotypes (1, 4, 5, 6B, 7F, 9V, 14, 18C, 19F, 23F), and PREVNAR 13™ contains conjugated saccharides from thirteen serotypes (1, 3, 4, 5, 6A, 6B, 7F, 9V, 14, 18C, 19A, 19F, and 23F). Saccharides are preferably conjugated to carrier proteins [*e.g*. refs. 62 to 64]. Typical carrier proteins are tetanus toxoid, diphtheria toxoid, the CRM197 derivative of diphtheria toxin, and *H. influenzae* protein D. Saccharides in the PREVNAR™ product are individually conjugated to CRM197 by reductive amination, with 2µg of each saccharide per 0.5ml dose (4µg of serotype 6B). SYNFLORIX™ uses three different carrier proteins and a mixture of different saccharide quantities for the different serogroups.

Quantities of pneumococcal antigens are typically expressed in µg of saccharide. The concentration of a pneumococcal conjugate, measured as saccharide, is typically between 2 and 20 µg/ml for each serotype.

### Hepatitis B virus

Hepatitis B virus (HBV) is a cause of viral hepatitis. The HBV virion consists of an inner core surrounded by an outer protein coat or capsid, and the core contains the viral DNA genome. The major component of the capsid is a protein known as HBV surface antigen or, more commonly, 'HBsAg', which is typically a 226-amino acid polypeptide with a molecular weight of ∼24 kDa. All existing hepatitis B vaccines contain HBsAg, and when this antigen is administered to a normal vaccinee, it stimulates the production of anti-HBsAg antibodies which protect against HBV infection. Thus a combination vaccine of the invention can include HBsAg.

For vaccine manufacture, HBsAg can be made in two ways. The first method involves purifying the antigen in particulate form from the plasma of chronic hepatitis B carriers, as large quantities of HBsAg are synthesized in the liver and released into the blood stream during an HBV infection. The second way involves expressing the protein by recombinant DNA methods. HBsAg for use with the method of the invention should be recombinantly expressed, *e.g.* in yeast cells. Suitable yeasts include *Saccharomyces* (such as *S.cerevisiae*), *Hanensula* (such as *H.polymorpha*) or *Pichia* hosts. The yeasts can be cultured in the absence of animal-derived components.

Unlike native HBsAg *(i.e.* as in the plasma-purified product), yeast-expressed HBsAg is generally non-glycosylated, and this is the most preferred form of HBsAg for use with the invention. Yeast-expressed HBsAg is highly immunogenic and can be prepared without the risk of blood product contamination. Many methods for purifying HBsAg from recombinant yeast are known in the art.

The HBsAg will generally be in the form of substantially-spherical particles (average diameter of about 20nm), including a lipid matrix comprising phospholipids. Yeast-expressed HBsAg particles may include phosphatidylinositol, which is not found in natural HBV virions. The particles may also include a non-toxic amount of LPS in order to stimulate the immune system [65]. The particles may retain non-ionic surfactant (*e.g*. polysorbate 20) if this was used during disruption of yeast [66].

The HBsAg is preferably from HBV subtype adw2.

A preferred method for HBsAg purification involves, after cell disruption: ultrafiltration; size exclusion chromatography; anion exchange chromatography; ultracentrifugation; desalting; and sterile filtration. Lysates may be precipitated after cell disruption (*e.g*. using a polyethylene glycol), leaving HBsAg in solution, ready for ultrafiltration.

After purification HBsAg may be subjected to dialysis (*e.g*. with cysteine), which can be used to remove any mercurial preservatives such as thimerosal that may have been used during HBsAg preparation [67].

Quantities of HBsAg are typically expressed in micrograms. Combination vaccines containing HBsAg usually include between 5 and 60 µg/ml. The concentration of HBsAg in a composition of the invention is preferably less than 60 µg/ml *e.g.* ≤55 µg/ml, ≤50 µg/ml, ≤45 µg/ml, ≤40 µg/ml, *etc.* A concentration of about 20 µg/ml is typical e.g. 10µg per dose. In some embodiments of the invention, a composition includes a 'low dose' of HBsAg. This means that the concentration of HBsAg in the composition is ≤5 µg/ml *e.g.* <4, <3, <2.5, <2, <1, *etc.* In a typical 0.5ml unit dose volume, therefore, the amount of HBsAg is less than 2.5µg *e.g.* <2, <1.5, <1, <0.5, *etc.*

### Poliovirus

Poliovirus causes poliomyelitis. Inactivated polio virus vaccine (IPV), as disclosed in more detail in chapter 24 of reference 1, has been known for many years. Thus a combination vaccine of the invention can include an inactivated poliovirus antigen.

Polioviruses may be grown in cell culture, and a preferred culture uses a Vero cell line, derived from monkey kidney. Vero cells can conveniently be cultured microcarriers. After growth, virions may be purified using techniques such as ultrafiltration, diafiltration, and chromatography. Where animal (and particularly bovine) materials are used in the culture of cells, they should be obtained from sources that are free from transmissible spongiform encephalopathies (TSEs), and in particular free from bovine spongiform encephalopathy (BSE). Preferably, polioviruses are grown in cells cultured in medium free of animal-derived components.

Prior to administration to patients, polioviruses must be inactivated, and this can be achieved by treatment with formaldehyde (or, preferably, a non-aldehyde agent). Poliomyelitis can be caused by one of three types of poliovirus. The three types are similar and cause identical symptoms, but they are antigenically very different and infection by one type does not protect against infection by others. It is therefore preferred to use three poliovirus antigens with the invention: poliovirus Type 1 (*e.g*. Mahoney strain), poliovirus Type 2 (*e.g.* MEF-1 strain), and poliovirus Type 3 (*e.g.* Saukett strain). The viruses are preferably grown, purified and inactivated individually, and are then combined to give a bulk trivalent mixture for use with the invention.

Quantities of IPV are typically expressed in the 'DU' unit (the "D-antigen unit" [68]). Combination vaccine usually comprise between 1-100 DU per polioviral type per dose *e.g*., about 40 DU of type 1 poliovirus, about 8 DU of type 2 poliovirus, and about 32 DU of type 3 poliovirus, but it is possible to use lower doses than these [69,70] *e.g.* 10-20 DU for type 1, 2-4 DU for type 2, and 8-20 DU for type 3. A combination vaccine of the invention can include a 'low dose' of a poliovirus. For a Type 1 poliovirus this means that the concentration of the virus in the composition is ≤20 DU/ml *e.g.* <18, <16, <14, <12, <10, *etc.* For a Type 2 poliovirus this means that the concentration of the virus in the composition is ≤4 DU/ml *e.g.* <3, <2, <1, <0.5, *etc.* For a Type 3 poliovirus this means that the concentration of the virus in the composition is ≤16 DU/ml *e.g.* <14, <12, <10, <8, <6, *etc.* Where all three of Types 1, 2 and 3 poliovirus are present the three antigens can be present at a DU ratio of 5:1:4 respectively, or at any other suitable ratio *e.g.* a ratio of 15:32:45 when using Sabin strains [71]. A low dose of antigen from Sabin strains is particularly useful, with ≤10 DU type 1, ≤20 DU type 2, and ≤30 DU type 3 (per unit dose, typically 0.5 ml).

Where an IPV component is used, and the polioviruses were grown on Vero cells, a vaccine composition preferably contains less than 10ng/ml, preferably ≤1ng/ml *e.g*. ≤500pg/ml or ≤50 pg/ml of Vero cell DNA *e.g.* less than 10ng/ml of Vero cell DNA that is ≥50 base pairs long.

### Preparing a combination vaccine

Antigenic components from these pathogens for use in vaccines are commonly referred to by abbreviated names: 'D' for diphtheria toxoid; 'T' for tetanus toxoid; 'P' for pertussis antigens, with 'aP' being acellular (*e.g.* including at least PT and optionally FHA and/or pertactin); 'Hib' for conjugated *H.influenzae* b capsular saccharide; 'MenA', 'MenB', 'MenC', 'MenW' and 'MenY' for the respective meningococcal serogroups, separately conjugated to carrier proteins; 'IPV' for 3-valent inactivated poliovirus; and 'Spn' for pneumococcus.

Embodiments of the invention include, but are not limited to combination vaccines comprising the following components:
- D, T, aP
- D, T, aP, IPV
- D, T, aP, HBsAg
- D, T, aP, Hib
- D, T, aP, Hib, IPV
- D, T, aP, HBsAg, Hib
- D, T, aP, HBsAg, IPV
- D, T, aP, HBsAg, IPV, Hib
- D, T, aP, HBsAg, IPV, Hib, Spn
- D, T, aP, HBsAg, IPV, Hib, MenC
- D, T, aP, HBsAg, IPV, Hib, MenC, MenA
- D, T, aP, HBsAg, IPV, Hib, MenC, MenY
- D, T, aP, HBsAg, IPV, Hib, MenC, MenW135
- D, T, aP, HBsAg, IPV, Hib, MenC, MenA, MenW135, MenY

These combination vaccines may consist of the antigens listed, or may further include antigens from additional pathogens. Thus they can be used separately, or as components of further vaccines.

For pediatric combination vaccines, the ratio of D:T is typically greater than 1 (*i.e.* pediatric vaccines usually have excess D in Lf units) and generally between 2:1 and 3:1 (measured in Lf units), *e.g.* 2.5:1. In contrast, for booster vaccine that are administered to adolescents or adults (who usually have received at least one paediatric combination vaccine comprising D and T), the ratio of T:D is typically greater than 1 (*i.e.* booster vaccines usually have excess T in Lf units) and generally between 1.5:1 and 2.5:1, *e.g.* 2:1. One useful vaccine includes (per unit dose) 2Lf D, 5Lf T, 4µg PT-9K/129G, 4µg FHA and 8µg pertactin. Another useful vaccine includes (per unit dose) 25Lf D, 10Lf T, 25µg PT-9K/129G, 25µg FHA and 8µg pertactin.

When combining antigenic components to prepare a multivalent composition, the antigens can be added individually, or they can be pre-mixed. Where a combination vaccine comprises D and T antigens and additional antigens, a pre-mixed D-T component can be used in the preparation of the combination vaccine. This bivalent component can be combined with further antigens. Where a D-T mixture is used for preparing a combination vaccines, the ratio of diphtheria toxoid to tetanus toxoid in the mixture can be between 2:1 and 3:1 (measured in Lf units), preferably between 2.4:1 and 2.6:1, *e.g*. preferably 2.5:1.

### Carrier proteins for conjugates

Conjugated saccharide antigens include a carrier protein, to which the saccharide is covalently attached, either directly or via a linker. General information on conjugation techniques can be found in reference 33.

Various proteins are known for use as carriers, and preferred carrier proteins are bacterial toxoids, such as diphtheria toxoid or tetanus toxoid. Other suitable carrier proteins include, but are not limited to, the CRM197 mutant of diphtheria toxin [52-54], the *N.meningitidis* outer membrane protein [72], synthetic peptides [73, 74], heat shock proteins [75,76], pertussis proteins [77,78], cytokines [79], lymphokines [79], hormones [79], growth factors [79], artificial proteins comprising multiple human CD4⁺ T cell epitopes from various pathogen-derived antigens [80] such as N19 [81], protein D from *H.influenzae* [82,83], pneumococcal surface protein PspA [84], pneumolysin [85], iron-uptake proteins [86], toxin A or B from *C.difficile* [87], *S.agalactiae* proteins [88], *etc.*

Attachment of a saccharide to a carrier is preferably via a -NH₂ group *e.g.* in the side chain of a lysine residue in a carrier protein, or of an arginine residue. Attachment to -SH groups (*e.g.* in the side chain of a cysteine) is also possible.

Conjugates with a saccharide:protein ratio (w/w) of between 1:5 (*i.e.* excess protein) and 5:1 (*i.e.* excess saccharide) are preferred.

Compositions may include a small amount of free carrier. Ignoring any carrier included as a separate antigen, unconjugated carrier is preferably no more than 5% of the total amount of the carrier protein in the composition as a whole, and more preferably present at less than 2% by weight.

As in the SYNFLORIX™ product, it is possible to include more than one type of carrier protein in a composition *e.g.* to reduce the risk of carrier suppression.

Amounts of conjugates are generally given in terms of mass of saccharide (*i.e.* the dose of the conjugate as a whole (*i.e.* carrier + saccharide) is higher than the stated dose) in order to avoid variation due to choice of carrier.

### Aluminium Salt Adjuvants

Vaccines of the invention will generally include an adjuvant. The most usual adjuvant for inclusion is an aluminium salt, such as an aluminium hydroxide and/or an aluminium phosphate. Antigens in a combination vaccine can be adsorbed (partially or totally) to aluminium salts.

The adjuvants commonly known as "aluminium hydroxide" are typically aluminium oxyhydroxide salts, which are usually at least partially crystalline. Aluminium oxyhydroxide, which can be represented by the formula AlO(OH), can be distinguished from other aluminium compounds, such as aluminium hydroxide Al(OH)₃, by infrared (IR) spectroscopy, in particular by the presence of an adsorption band at 1070cm⁻¹ and a strong shoulder at 3090-3100cm⁻¹ (chapter 9 of reference 89). The degree of crystallinity of an aluminium hydroxide adjuvant is reflected by the width of the diffraction band at half height (WHH), with poorly-crystalline particles showing greater line broadening due to smaller crystallite sizes. The surface area increases as WHH increases, and adjuvants with higher WHH values have been seen to have greater capacity for antigen adsorption. A fibrous morphology (*e.g*. as seen in transmission electron micrographs) is typical for aluminium hydroxide adjuvants *e.g*. with needle-like particles with diameters about 2nm. The pI of aluminium hydroxide adjuvants is typically about 11 *i.e.* the adjuvant itself has a positive surface charge at physiological pH. Adsorptive capacities of between 1.8-2.6 mg protein per mg Al⁺⁺⁺ at pH 7.4 have been reported for aluminium hydroxide adjuvants.

The adjuvants commonly known as "aluminium phosphate" are typically aluminium hydroxyphosphates, often also containing a small amount of sulfate (*i.e.* aluminium hydroxyphosphate sulfate). They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 1.2. Hydroxyphosphates can be distinguished from strict AlPO₄ by the presence of hydroxyl groups. For example, an IR spectrum band at 3164cm⁻¹ (*e.g.* when heated to 200°C) indicates the presence of structural hydroxyls (chapter 9 of reference 89). The PO₄/Al³⁺ molar ratio of an aluminium phosphate adjuvant will generally be between 0.3 and 1.2, preferably between 0.8 and 1.2, and more preferably 0.95±0.1. The aluminium phosphate will generally be amorphous, particularly for hydroxyphosphate salts. A typical adjuvant is amorphous aluminium hydroxyphosphate with PO₄/Al molar ratio between 0.84 and 0.92, included at 0.6mg Al³⁺/ml. The aluminium phosphate will generally be particulate (*e.g*. plate-like morphology as seen in transmission electron micrographs, with primary particles in the range of 50nm). Typical diameters of the particles are in the range 0.5-20µm (*e.g*. about 5-10µm) after any antigen adsorption. Adsorptive capacities of between 0.7-1.5 mg protein per mg Al⁺⁺⁺ at pH 7.4 have been reported for aluminium phosphate adjuvants.

The PZC of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 *e.g.* about 5.7.

The concentration of Al⁺⁺⁺ in a composition for administration to a patient is preferably less than 10mg/ml *e.g.* ≤5 mg/ml, ≤4 mg/ml, ≤3 mg/ml, ≤2 mg/ml, ≤1 mg/ml, *etc.* A preferred range of Al⁺⁺⁺ in a composition of the invention is between 0.3 and 1mg/ml or between 0.3-0.5mg/ml. A maximum of 0.85mg/dose is typical.

In the vaccine composition of the invention, the *B. pertussis* antigens may be adsorbed onto one or more aluminium salt adjuvants, or may be added in an unadsorbed state. Where pertactin is present in a composition of the invention, then it is preferably adsorbed onto an aluminium hydroxide adjuvant. PT and FHA may be adsorbed onto an aluminium hydroxide adjuvant or an aluminium phosphate in a composition of the invention. In preferred embodiments, PT, FHA and pertactin are adsorbed to aluminium hydroxide.

In a combination vaccine comprising a diphtheria toxoid, the diphtheria toxoid is adsorbed onto an aluminium salt adjuvant e.g. is adsorbed to an aluminium hydroxide adjuvant or an aluminium phosphate adjuvant.

In a combination vaccine comprising a tetanus toxoid, the tetanus toxoid may be adsorbed onto an aluminium hydroxide adjuvant, but this is not necessary (*e.g*. adsorption of between 0-10% of the total tetanus toxoid can be used). Alternatively, the tetanus toxoid may be adsorbed onto an aluminium phosphate adjuvant.

In a combination vaccine comprising Hib antigens and an aluminium salt, the Hib conjugate may be unadsorbed or can be adsorbed (*e.g*. adsorbed to an aluminium phosphate adjuvant [90]). Adsorption in this way is particularly useful in vaccines comprising D-T-Pw-Hib-HBsAg antigens. Other conjugated antigens (*e.g.* meningococcus, pneumococcus) can similarly be adsorbed to an aluminium salt (*e.g.* a phosphate) or can be unadsorbed [91].

IPV antigens need not be adsorbed to any adjuvant before being mixed with other components of a combination vaccine, but they can become adsorbed onto aluminium adjuvant(s) originating from these other components.

In a combination vaccine comprising HBsAg, the HBsAg can be adsorbed onto aluminium phosphate using the methods described in reference 92. Adsorption to aluminium phosphate contrasts with the well-known ENGERIX-B™ product (where HBsAg is adsorbed to aluminium hydroxide). As mentioned in reference 93, aluminium phosphate can be a better adjuvant for HBsAg than aluminium hydroxide.

Where a process of the invention utilises a component in which diphtheria and tetanus toxoids have been mixed prior to their being combined with HBsAg, this D-T mixture preferably contains an aluminium hydroxide adjuvant, to which the D and T antigens are both adsorbed.

When an adjuvant is included in a vaccine of the invention, it can be added at various stages. Antigens can be combined with adjuvants before being used in preparing combination vaccines (*e.g*. a bivalent D-T mixture can be adsorbed to aluminium salt adjuvant(s) before being used in a process of the invention), but it is also possible to add adjuvant after the antigens have been mixed, or to add a sequence of antigens to an adjuvant (*e.g.* to start with an aqueous adjuvant, then to add antigens, either individually or pre-mixed).

### Oil-in-water emulsion adjuvants

Compositions of the invention may include an oil-in-water emulsion adjuvant.

Various such emulsions are known *e.g*. MF59 and AS03 are both authorised in Europe.

Useful emulsion adjuvants they typically include at least one oil and at least one surfactant, with the oil(s) and surfactant(s) being biodegradable (metabolisable) and biocompatible. The oil droplets in the emulsion generally have a sub-micron diameter, and these small sizes can readily be achieved with a microfluidiser to provide stable emulsions, or by alternative methods *e.g.* phase inversion. Emulsions in which at least 80% (by number) of droplets have a diameter of less than 220nm are preferred, as they can be subjected to filter sterilization.

The emulsion can include oil(s) from an animal (such as fish) and/or vegetable source. Sources for vegetable oils include nuts, seeds and grains. Peanut oil, soybean oil, coconut oil, and olive oil, the most commonly available, exemplify the nut oils. Jojoba oil can be used e.g. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolisable and may therefore be used with the invention. The procedures for separation, purification, saponification and other means necessary for obtaining pure oils from animal sources are well known in the art.

Most fish contain metabolisable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred for use with the invention (see below). Squalane, the saturated analog to squalene, is also a useful oil. Fish oils, including squalene and squalane, are readily available from commercial sources or may be obtained by methods known in the art. Other preferred oils are the tocopherols (see below). Mixtures of oils can be used.

Preferred amounts of total oil (% by volume) in an adjuvant emulsion are between 1 and 20% e.g. between 2-10%. A squalene content of 5% by volume is particularly useful.

Surfactants can be classified by their 'HLB' (hydrophile/lipophile balance). Preferred surfactants used with the invention have a HLB of at least 10 *e.g.* about 15. The invention can be used with surfactants including, but not limited to: the polyoxyethylene sorbitan esters surfactants (commonly referred to as the Tweens), especially polysorbate 20 or polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the Spans), such as sorbitan trioleate (Span 85) or sorbitan monolaurate.

Emulsions used with the invention preferably include non-ionic surfactant(s). Preferred surfactants for including in the emulsion are polysorbate 80 (polyoxyethylene sorbitan monooleate; Tween 80), Span 85 (sorbitan trioleate), lecithin or Triton X-100. Mixtures of surfactants can be used *e.g.* a mixture of polysorbate 80 and sorbitan trioleate. A combination of a polyoxyethylene sorbitan ester such as polysorbate 80 (Tween 80) and an octoxynol such as t-octylphenoxypolyethoxyethanol (Triton X-100) is also useful. Another useful combination comprises laureth 9 plus a polyoxyethylene sorbitan ester and/or an octoxynol. Where a mixture of surfactants is used then the HLB of the mixture is calculated according to their relative weightings (by volume) *e.g*. the preferred 1:1 mixture by volume of polysorbate 80 and sorbitan trioleate has a HLB of 8.4.

Preferred amounts of total surfactant (% by volume) in an adjuvant emulsion are between 0.1 and 2% *e.g.* between 0.25-2%. A total content of 1% by volume is particularly useful *e.g.* 0.5% by volume of polysorbate 80 and 0.5% by volume of sorbitan trioleate.

Useful emulsions can be prepared using known techniques *e.g.* see references 94 to 101.

Specific oil-in-water emulsion adjuvants useful with the invention include, but are not limited to:
- A submicron emulsion of squalene, polysorbate 80, and sorbitan trioleate. The composition of the emulsion by volume can be about 5% squalene, about 0.5% polysorbate 80 and about 0.5% sorbitan trioleate. In weight terms, these ratios become 4.3% squalene, 0.5% polysorbate 80 and 0.48% sorbitan trioleate. This adjuvant is known as 'MF59' [102-104], as described in more detail in Chapter 10 of ref. 89 and chapter 12 of ref. 94. The MF59 emulsion advantageously includes citrate ions *e.g.* 10mM sodium citrate buffer.
- An emulsion of squalene, a tocopherol, and polysorbate 80. The emulsion may include phosphate buffered saline. These emulsions may have from 2 to 10% squalene, from 2 to 10% tocopherol and from 0.3 to 3% polysorbate 80, and the weight ratio of squalene:tocopherol is preferably ≤1 (*e.g.* 0.90) as this can provide a more stable emulsion. Squalene and polysorbate 80 may be present volume ratio of about 5:2, or at a weight ratio of about 11:5. Thus the three components (squalene, tocopherol, polysorbate 80) may be present at a weight ratio of 1068:1186:485 or around 55:61:25. This adjuvant is known as 'AS03'. Another useful emulsion of this type may comprise, per human dose, 0.5-10 mg squalene, 0.5-11 mg tocopherol, and 0.1-4 mg polysorbate 80 [105] *e.g.* in the ratios discussed above.
- An emulsion in which a saponin (*e.g.* QuilA or QS21) and a sterol (*e.g.* a cholesterol) are associated as helical micelles [106].
- An emulsion having from 0.5-50% of an oil, 0.1-10% of a phospholipid, and 0.05-5% of a non-ionic surfactant. As described in reference 107, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- An emulsion comprising squalene, an aqueous solvent, a polyoxyethylene alkyl ether hydrophilic nonionic surfactant (*e.g*. polyoxyethylene (12) cetostearyl ether) and a hydrophobic nonionic surfactant (*e.g*. a sorbitan ester or mannide ester, such as sorbitan monoleate or 'Span 80'). The emulsion is preferably thermoreversible and/or has at least 90% of the oil droplets (by volume) with a size less than 200 nm [108]. The emulsion may also include one or more of: alditol; a cryoprotective agent (*e.g.* a sugar, such as dodecylmaltoside and/or sucrose); and/or an alkylpolyglycoside. It may also include a TLR4 agonist, such as one whose chemical structure does not include a sugar ring [109]. Such emulsions may be lyophilized. The 'AF03' product is one such emulsion.

Preferred oil-in-water emulsions used with the invention comprise squalene and polysorbate 80.

The emulsions may be mixed with antigens during vaccine manufacture, or they may be mixed extemporaneously at the time of delivery. Thus, in some embodiments, the adjuvant and antigens may be kept separately in a packaged or distributed vaccine, ready for final formulation at the time of use. At the time of mixing (whether during bulk manufacture, or at the point of use) the antigen will generally be in an aqueous form, such that the final vaccine is prepared by mixing two liquids. The volume ratio of the two liquids for mixing can vary (*e.g*. between 5:1 and 1:5) but is generally about 1:1. If emulsion and antigen are stored separately in a kit then the product may be presented as a vial containing emulsion and a vial containing aqueous antigen, for mixing to give adjuvanted liquid vaccine (monodose or multi-dose).

Preferred emulsions of the invention include squalene oil. This is usually prepared from shark oil but alternative sources are known *e.g*. see references 110 (yeast) and 111 (olive oil). Squalene which contains less than 661 picograms of PCBs per gram of squalene (TEQ) is preferred for use with the invention, as disclosed in reference 112. The emulsions are preferably made from squalene of high purity *e.g*. prepared by double-distillation as disclosed in reference 113.

Where a composition includes a tocopherol, any of the α, β, γ, δ, ε or ξ tocopherols can be used, but α-tocopherols are preferred. The tocopherol can take several forms *e.g*. different salts and/or isomers. Salts include organic salts, such as succinate, acetate, nicotinate, *etc.* D-α-tocopherol and DL-α-tocopherol can both be used. Tocopherols have antioxidant properties that may help to stabilize the emulsions [114]. A preferred α-tocopherol is DL-α-tocopherol, and a preferred salt of this tocopherol is the succinate.

### TLR agonists

Compositions of the invention may include a TLR agonist, *i.e.* a compound which can agonise a Toll-like receptor. Most preferably, a TLR agonist is an agonist of a human TLR. The TLR agonist can activate any of TLR1, TLR2, TLR3, TLR4, TLR5, TLR6, TLR7, TLR8, TLR9 or TLR11; preferably it can activate human TLR4 or human TLR7.

Agonist activity of a compound against any particular Toll-like receptor can be determined by standard assays. Companies such as Imgenex and Invivogen supply cell lines which are stably co-transfected with human TLR genes and NFκB, plus suitable reporter genes, for measuring TLR activation pathways. They are designed for sensitivity, broad working range dynamics and can be used for high-throughput screening. Constitutive expression of one or two specific TLRs is typical in such cell lines. See also reference 115. Many TLR agonists are known in the art *e.g*. reference 116 describes certain lipopeptide molecules that are TLR2 agonists, references 117 to 120 each describe classes of small molecule agonists of TLR7, and references 121 and 122 describe TLR7 and TLR8 agonists for treatment of diseases.

It is possible to adsorb TLR agonists to aluminium salts, thereby improving the immunopotentiating effect of the adjuvant [123]. This can lead to a better (stronger, or more quickly achieved) immune response and/or permits a reduction in the amount of aluminium in the composition while maintaining an equivalent adjuvant effect. A composition of the invention can therefore include an aluminium salt to which the TLR agonist is adsorbed. The agonist and the salt can form a stable adjuvant complex which retains (at least in part) the salt's ability to adsorb antigens.

Phosphorus-containing adsorptive moieties are particularly useful, and so an adsorptive moiety may comprise a phosphate, a phosphonate, a phosphinate, a phosphonite, a phosphinite, *etc.* These groups can undergo ligand exchange with surface groups on an aluminium salt, and particularly with a salt having surface hydroxide groups.

In preferred embodiments, a composition of the invention includes a TLR agonist (more preferably a TLR7 agonist) which includes a phosphonate group. This phosphonate group can allow adsorption of the agonist to an insoluble aluminium salt [123].

TLR agonists useful with the invention may include a single adsorptive moiety, or may include more than one *e.g*. between 2 and 15 adsorptive moieties. Typically a compound will include 1, 2 or 3 adsorptive moieties.

Phosphorus-containing TLR agonists useful with the invention can be represented by formula (A1): 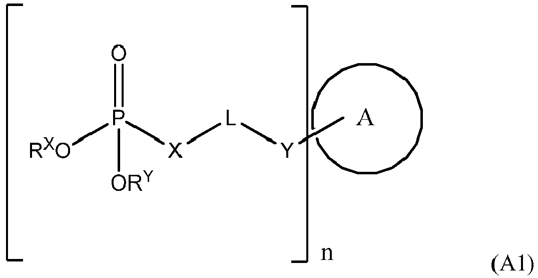 wherein:
R^{X} and R^{Y} are independently selected from H and C₁-C₆ alkyl;
X is selected from a covalent bond, O and NH;
Y is selected from a covalent bond, O, C(O), S and NH;
L is a linker *e.g*. selected from, C₁-C₆alkylene, C₁-C₆alkenylene, arylene, heteroarylene, C₁-C₆alkyleneoxy and -((CH₂)ₚO)_{q}(CH₂)ₚ- each optionally substituted with 1 to 4 substituents independently selected from halo, OH, C₁-C₄alkyl, -OP(O)(OH)₂ and -P(O)(OH)₂;
each p is independently selected from 1, 2, 3, 4, 5 and 6;
q is selected from 1, 2, 3 and 4;
n is selected from 1, 2 and 3; and
A is a TLR agonist moiety.

In one embodiment, the TLR agonist according to formula (A1) is as follows: R^{X} and R^{Y} are H; X is O; L is selected from C₁-C₆ alkylene and -((CH2)ₚO)_{q}(CH2)ₚ- each optionally substituted with 1 to 2 halogen atoms; p is selected from 1, 2 and 3; q is selected from 1 and 2; and n is 1. Thus in these embodiments the adsorptive moiety comprises a phosphate group.

In other embodiments, the TLR agonist according to formula (A1) is as follows: R^{X} and R^{Y} are H; X is a covalent bond; L is selected from C₁-C₆ alkylene and -((CH2)ₚO)_{q}(CH2)ₚ- each optionally substituted with 1 to 2 halogen atoms; p is selected from 1, 2 or 3; q is selected from 1 or 2; and n is 1. Thus in these embodiments the adsorptive moiety comprises a phosphonate group.

In some embodiments, the TLR agonist moiety 'A' has a molecular weight of less than 1000 Da. In some embodiments, the TLR agonist of formula (A1) has a molecular weight of less than 1000 Da.

Preferred TLR agonists are water-soluble. Thus they can form a homogenous solution when mixed in an aqueous buffer with water at pH 7 at 25°C and 1 atmosphere pressure to give a solution which has a concentration of at least 50µg/ml. The term "water-soluble" thus excludes substances that are only sparingly soluble under these conditions.

Useful TLR agonists include those having formula (B), (C), (D), (E), (F), (G), (H), (I), (II), (IV), or (J) as described in reference 123. Other useful TLR agonists are compounds 1 to 102 as defined in reference 123. Preferred TLR7 agonists have formula (IV) from reference 123, such as compound K1 or K2 identified below. These can be used as salts *e.g*. the arginine salt of K2.

The amount of TLR agonist in a unit dose will fall in a relatively broad range that can be determined through routine trials. An amount of between 1-1000µg/dose can be used *e.g*. from 5-100µg per dose or from 10-100µg per dose, and ideally ≤300µg per dose *e.g*. about 5µg, 10µg, 20µg, 25µg, 50µg or 100µg per dose. Thus the concentration of a TLR agonist in a composition of the invention may be from 2-2000µg/ml *e.g*. from 10-200µg/ml, or about 5, 10, 20, 40, 50, 100 or 200µg/ml, and ideally ≤600µg/ml.

In general, the weight ratio of TLR agonist to Al⁺⁺⁺ in a composition will be less than 5:1 *e.g.* less than 4:1, less than 3:1, less than 2:1, or less than 1:1. Thus, for example, with an Al⁺⁺⁺ concentration of 0.5mg/ml the maximum concentration of TLR agonist would be 2.5mg/ml. But higher or lower levels can be used. A lower mass of TLR agonist than of Al⁺⁺⁺ can be most typical *e.g.* per dose, 100µg of TLR agonist with 0.2mg Al⁺⁺⁺, *etc.* For instance, the *Fendrix* product includes 50µg of 3d-MPL and 0.5mg Al⁺⁺⁺ per dose.

It is preferred that at least 50% (by mass) of a TLR agonist in the composition is adsorbed to the aluminium salt *e.g.* ≥60%, ≥70%, ≥80%, ≥85%, ≥90%, ≥92%, ≥94%, ≥95%, ≥96%, ≥97%, ≥98%, ≥99%, or even 100%.

Where a composition of the invention includes a TLR agonist adsorbed to a metal salt, and also includes a buffer, it is preferred that the concentration of any phosphate ions in the buffer should be less than 50mM (*e.g.* between 1-15mM) as a high concentration of phosphate ions can cause desorption. Use of a histidine buffer is preferred.

### Toll-like receptor 4 agonists

Compositions of the invention can include a TLR4 agonist, preferably an agonist of human TLR4. TLR4 is expressed by cells of the innate immune system, including conventional dendritic cells and macrophages [124]. Triggering via TLR4 induces a signalling cascade that utilizes both the MyD88- and TRIF-dependent pathways, leading to NF-κB and IRF3/7 activation, respectively. TLR4 activation typically induces robust IL-12p70 production and strongly enhances Th1-type cellular and humoral immune responses.

Various useful TLR4 agonists are known in the art, many of which are analogs of endotoxin or lipopolysaccharide (LPS). For instance, the TLR4 agonist can be:
(i) 3d-MPL (*i.e.* 3-O-deacylated monophosphoryl lipid A; also known as 3-de-O-acylated monophosphoryl lipid A or 3-O-desacyl-4'-monophosphoryl lipid A). This derivative of the monophosphoryl lipid A portion of endotoxin has a de-acylated position 3 of the reducing end of glucosamine. It has been prepared from a heptoseless mutant of *Salmonella minnesota*, and is chemically similar to lipid A but lacks an acid-labile phosphoryl group and a base-labile acyl group. Preparation of 3d-MPL was originally described in ref. 125, and the product has been manufactured and sold by Corixa Corporation. It is present in GSK's 'AS04' adjuvant. Further details can be found in references 126 to 129.
(ii) an aminoalkyl glucosaminide phosphate, such as RC-529 or CRX-524 [130-132]. RC-529 and CRX-524 have the following structure, differing by their R₂ groups: 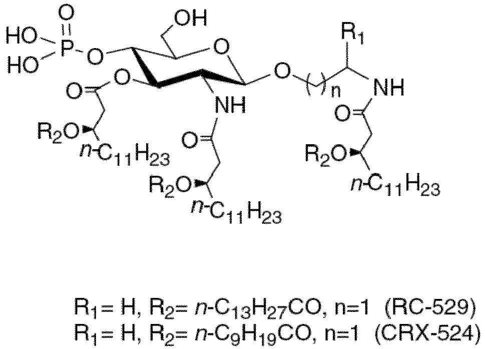
(iii) compounds containing lipids linked to a phosphate-containing acyclic backbone, such as the TLR4 antagonist E5564 [133,134]: 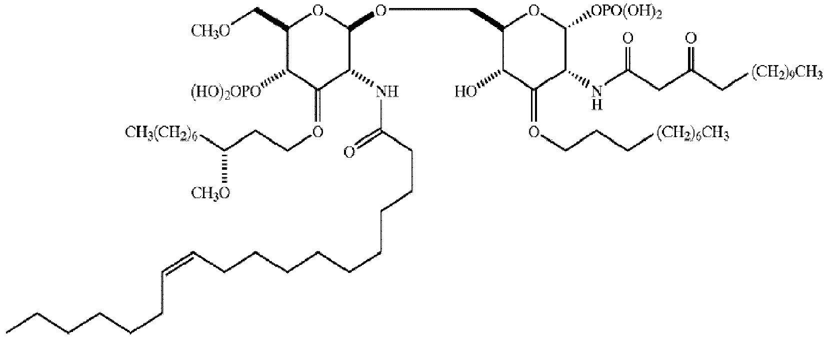
(iv) glucopyranosyl lipid A (GLA) [135] or its ammonium salt *e.g.* 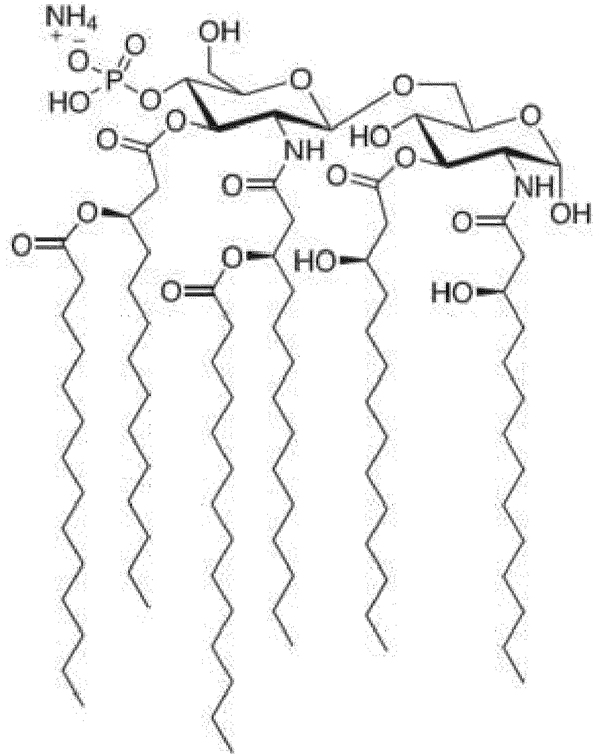
(v) A compound of formula I, II or III as defined in reference 136, or a salt thereof, such as compounds 'ER 803058', 'ER 803732', 'ER 804053', 'ER 804058', 'ER 804059', 'ER 804442', 'ER 804680', 'ER 803022', 'ER 804764' or 'ER 804057'. ER 804057 is also known as E6020 and it has the following structure: 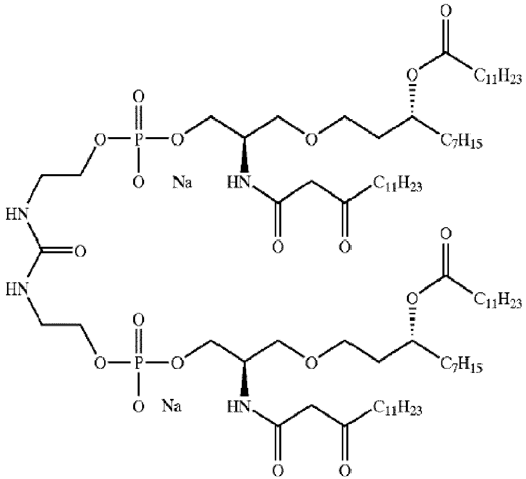 whereas ER 803022 has the following structure: 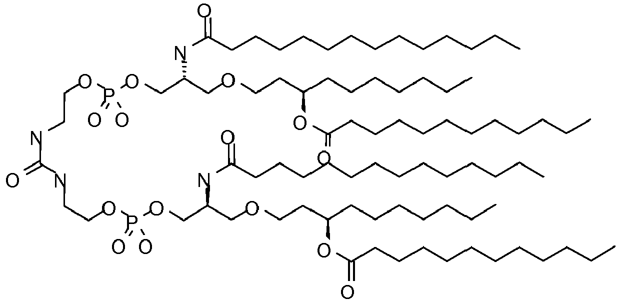
(vi) One of the polypeptide ligands disclosed in reference 137.

Any of these TLR4 agonists can be used with the invention.

A preferred TLR4 agonist for use with the invention is 3d-MPL. This can be adsorbed to an aluminium phosphate adjuvant, to an aluminium hydroxide adjuvant, or to a mixture of both [138].

3d-MPL can take the form of a mixture of related molecules, varying by their acylation (*e.g*. having 3, 4, 5 or 6 acyl chains, which may be of different lengths). The two glucosamine (also known as 2-deoxy-2-amino-glucose) monosaccharides are N-acylated at their 2-position carbons (*i.e*. at positions 2 and 2'), and there is also O-acylation at the 3' position. The group attached to carbon 2 has formula -NH-CO-CH₂-CR¹R^{1'}. The group attached to carbon 2' has formula -NH-CO-CH₂-CR²R^{2'}. The group attached to carbon 3' has formula -O-CO-CH₂-CR³R^{3'}. A representative structure is:

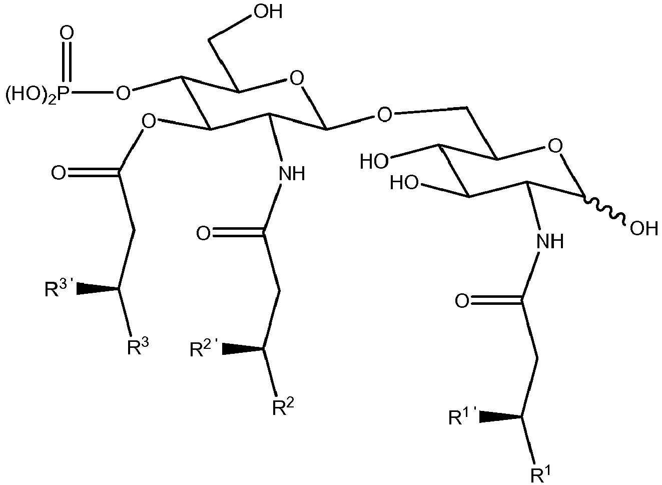

Groups R¹, R² and R³ are each independently -(CH₂)ₙ-CH₃. The value of *n* is preferably between 8 and 16, more preferably between 9 and 12, and is most preferably 10.

Groups R^{1'}, R^{2'} and R^{3'} can each independently be: (a) -H; (b) -OH; or (c) -O-CO-R⁴,where R⁴ is either - H or -(CH₂)ₘ-CH₃, wherein the value of *m* is preferably between 8 and 16, and is more preferably 10, 12 or 14. At the 2 position, *m* is preferably 14. At the 2' position, *m* is preferably 10. At the 3' position, *m* is preferably 12. Groups R^{1'}, R^{2'} and R^{3'} are thus preferably -O-acyl groups from dodecanoic acid, tetradecanoic acid or hexadecanoic acid.

When all of R^{1'}, R^{2'} and R^{3'} are -H then the 3d-MPL has only 3 acyl chains (one on each of positions 2, 2' and 3'). When only two of R^{1'}, R^{2'} and R^{3'} are -H then the 3d-MPL can have 4 acyl chains. When only one of R^{1'}, R^{2'} and R^{3'} is -H then the 3d-MPL can have 5 acyl chains. When none of R^{1'}, R^{2'} and R^{3'} is -H then the 3d-MPL can have 6 acyl chains. The 3d-MPL used according to the invention can be a mixture of these forms, with from 3 to 6 acyl chains, but it is preferred to include 3d-MPL with 6 acyl chains in the mixture, and in particular to ensure that the 6 acyl chain form makes up at least 10% by weight of the total 3d-MPL *e.g.* ≥20%, ≥30%, ≥40%, ≥50% or more. 3d-MPL with 6 acyl chains has been found to be the most adjuvant-active form.

Thus the most preferred form of 3d-MPL for use with the invention is:

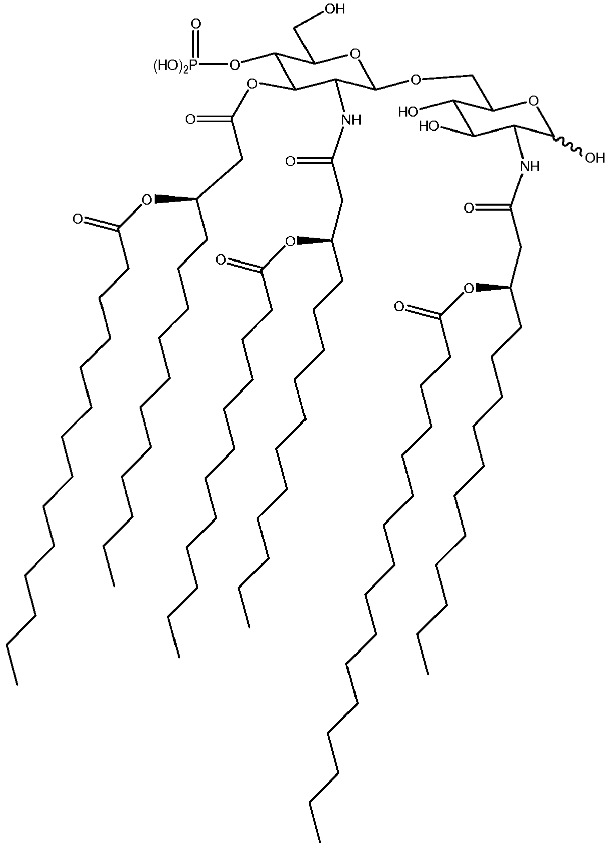

Where 3d-MPL is used in the form of a mixture then references to amounts or concentrations of 3d-MPL in compositions of the invention refer to the combined 3d-MPL species in the mixture.

Typical compositions include 3d-MPL at a concentration of between 25µg/ml and 200µg/ml *e.g*. in the range 50-150µg/ml, 75-125µg/ml, 90-110µg/ml, or about 100µg/ml. It is usual to administer between 25-75µg of 3d-MPL per dose *e.g.* between 45-55µg, or about 50µg 3d-MPL per dose.

In aqueous conditions, 3d-MPL can form micellar aggregates or particles with different sizes *e.g*. with a diameter <150nm or >500nm. Either or both of these can be used with the invention, and the better particles can be selected by routine assay. Smaller particles (*e.g*. small enough to give a clear aqueous suspension of 3d-MPL) are preferred for use according to the invention because of their superior activity [139]. Preferred particles have a mean diameter less than 150nm, more preferably less than 120nm, and can even have a mean diameter less than 100nm. In most cases, however, the mean diameter will not be lower than 50nm. Where 3d-MPL is adsorbed to an aluminum salt then it may not be possible to measure the 3D-MPL particle size directly, but particle size can be measured before adsorption takes place. Particle diameter can be assessed by the routine technique of dynamic light scattering, which reveals a mean particle diameter. Where a particle is said to have a diameter of *x* nm, there will generally be a distribution of particles about this mean, but at least 50% by number (e.g. ≥60%, ≥70%, ≥80%, ≥90%, or more) of the particles will have a diameter within the range *x*±25%.

### Toll-like receptor 7 agonists

Compositions of the invention can include a TLR7 agonist, preferably an agonist of human TLR7. The TLR agonist can be a compound according to formula (K): 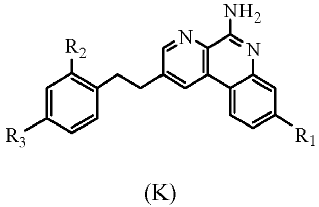 wherein:
R¹ is H, C₁-C₆alkyl, -C(R⁵)₂OH, -L¹R⁵, -L¹R⁶, -L²R⁵, -L²R⁶, -OL²R⁵, or -OL²R⁶,
L¹ is -C(O)- or -O-;
L² is C₁-C₆alkylene, C₂-C₆alkenylene, arylene, heteroarylene or -((CR⁴R⁴)ₚO)_{q}(CH₂)ₚ, wherein the C₁-C₆alkylene and C₂-C₆alkenylene of L² are optionally substituted with 1 to 4 fluoro groups;
each L³ is independently selected from C₁-C₆alkylene and -((CR⁴R⁴)ₚO)_{q}(CH₂)ₚ-, wherein the C₁-C₆alkylene of L³ is optionally substituted with 1 to 4 fluoro groups;
L⁴ is arylene or heteroarylene;
R² is H or C₁-C₆alkyl;
R³ is selected from C₁-C₄alkyl, -L³R⁵, -L¹R⁵, -L³R⁷, -L³L⁴L³R⁷, -L³L⁴R⁵, -L³L⁴L³R⁵, - OL³R⁵ , -OL³R⁷, -OL³L⁴R⁷, -OL³L⁴L³R⁷, -OR⁸, -OL³L⁴R⁵, -OL³L⁴L³R⁵ and -C(R⁵)₂OH ;
each R⁴ is independently selected from H and fluoro;
R⁵ is -P(O)(OR⁹)₂,
R⁶ is -CF₂P(O)(OR⁹)₂ or -C(O)OR¹⁰;
R⁷ is -CF₂P(O)(OR⁹)₂ or -C(O)OR¹⁰;
R⁸ is H or C₁-C₄alkyl;
each R⁹ is independently selected from H and C₁-C₆alkyl;
R¹⁰ is H or C₁-C₄alkyl;
each p is independently selected from 1, 2, 3, 4, 5 and 6, and
q is 1, 2, 3 or 4.

The compound of formula (K) is preferably of formula (K'): 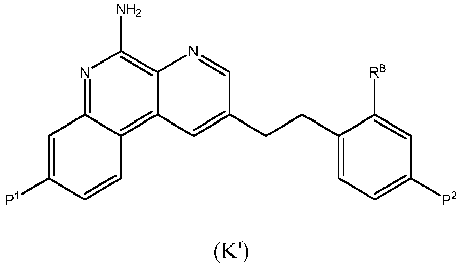 wherein:
P¹ is selected from H, C₁-C₆alkyl optionally substituted with COOH and -Y-L-X-P(O)(OR^{X})(OR^{Y});
P² is selected from H, C₁-C₆alkyl, C₁-C₆alkoxy and -Y-L-X-P(O)(OR^{X})(OR^{Y}); with the proviso that at least one of P¹ and P² is -Y-L-X-P(O)(OR^{X})(OR^{Y});
R^{B} is selected from H and C₁-C₆alkyl;
R^{X} and R^{Y} are independently selected from H and C₁-C₆alkyl;
X is selected from a covalent bond, O and NH;
Y is selected from a covalent bond, O, C(O), S and NH;
L is selected from, a covalent bond C₁-C₆alkylene, C₁-C₆alkenylene, arylene, heteroarylene, C₁-C₆alkyleneoxy and -((CH₂)ₚO)_{q}(CH₂)ₚ- each optionally substituted with 1 to 4 substituents independently selected from halo, OH, C₁-C₄alkyl, -OP(O)(OH)₂ and -P(O)(OH)₂;
each p is independently selected from 1, 2, 3, 4, 5 and 6; and
q is selected from 1, 2, 3 and 4.

In some embodiments of formula (K'): P¹ is selected from C₁-C₆alkyl optionally substituted with COOH and -Y-L-X-P(O)(OR^{X})(OR^{Y}); P² is selected from C₁-C₆alkoxy and -Y-L-X-P(O)(OR^{X})(OR^{Y}); R^{B} is C₁-C₆alkyl; X is a covalent bond; L is selected from C₁-C₆alkylene and -((CH₂)ₚO)_{q}(CH₂)ₚ- each optionally substituted with 1 to 4 substituents independently selected from halo, OH, C₁-C₄alkyl, -OP(O)(OH)₂ and-P(O)(OH)₂; each p is independently selected from 1, 2 and 3; q is selected from 1 and 2.

One useful TLR7 agonist is 'compound K1' (compound 6A on page 80 of reference 140):

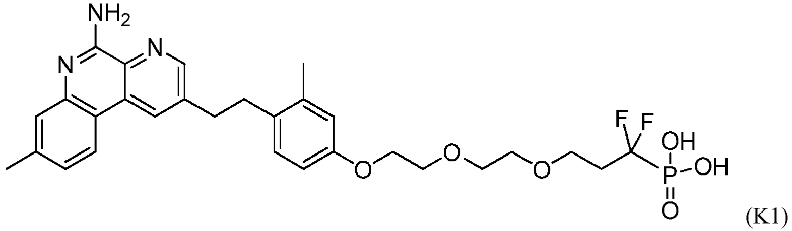

Compound K1 has a solubility of about 4mg/ml in water and adsorbs well to aluminium hydroxide.

Another preferred TLR7 agonist is compound 'K2' (compound 21A on page 83 of reference 140):

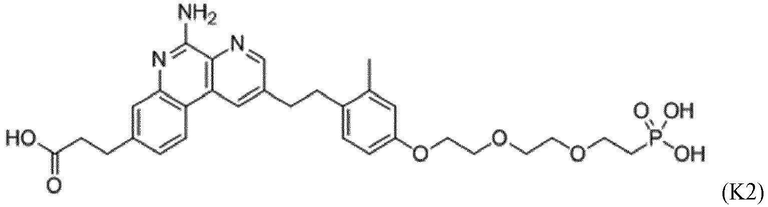

These TLR7 agonists can be used as salts *e.g*. the arginine salt of formula (K2), such as the arginine salt monohydrate

### Further non-antigen components

Vaccine compositions of the invention may comprise carriers, excipients, buffers, *etc.*

To control tonicity, a composition may include a physiological salt, such as a sodium salt. Sodium chloride (NaCl) is preferred, which may be present at between 1 and 20 mg/ml. In a specific embodiment, the sodium chloride concentration is between 8 and 9 mg/ml (*e.g*. about 8.5 mg/ml).

Compositions will generally have an osmolality of between 200 mOsm/kg and 400 mOsm/kg, preferably between 240-360 mOsm/kg, and will more preferably fall within the range of 280-320 mOsm/kg. Osmolality has previously been reported not to have an impact on pain caused by vaccination [141], but keeping osmolality in this range is nevertheless preferred.

Compositions of the invention may include one or more buffer(s). Typical buffers include: a phosphate buffer; a Tris buffer; a borate buffer; a succinate buffer; a histidine buffer; or a citrate buffer. Buffers will typically be included in the 5-20mM range.

Compositions of the invention may include one or more preservative(s), but in some embodiments the compositions are preservative-free. Due to the adsorbed nature of antigens, a vaccine product may be a suspension with a cloudy appearance. This appearance means that microbial contamination is not readily visible, and so the vaccine preferably contains a preservative. This is particularly important when the vaccine is packaged in multidose containers. Preferred preservatives for inclusion are 2-phenoxyethanol and thimerosal. It is preferred, however, not to use mercurial preservatives (*e.g*. thimerosal) during the process of the invention. Thus, between one and all of the components used in the process may be substantially free from mercurial preservative. However, the presence of trace amounts may be unavoidable if a component was treated with such a preservative before being used in the invention. For safety, it is preferred that the final composition contains less than about 25 ng/ml mercury. More preferably, the final vaccine product contains no detectable thimerosal. This will generally be achieved by removing the mercurial preservative from an antigen preparation prior to its addition in the process of the invention or by avoiding the use of thimerosal during the preparation of the components used to make the composition. Mercury-free compositions are preferred.

Compositions of the invention may further comprise a surfactant. Surfactants are added to reduce aggregation of antigen components. Precipitation of one or more antigen may occur during long-term storage of vaccine component in an aqueous solution. Addition of a surfactant such polysorbate 20 (polyoxyethylene (20) sorbitan monolaurate; Tween 20) and polysorbate 80 (polyoxyethylene (80) sorbitan monooleate; Tween 80) may reduce or prevent precipitation of antigens *e.g*. from a liquid combination vaccine. Preferably, the concentration of the surfactant does not exceed 0.05% by volume. Where a composition includes HBsAg, it will usually include polysorbate 20 *e.g.* if it was used during yeast disruption [66].

The pH of a composition of the invention will generally be between 5.0 and 7.5, and more typically between 5.0 and 6.0 for optimum stability or, where a diphtheria toxoid and/or tetanus toxoid is present, between 6.0 and 7.0. A process of the invention may therefore include a step of adjusting the pH of the bulk vaccine prior to packaging.

Compositions of the invention are preferably non-pyrogenic *e.g*. containing <1 EU (endotoxin unit, a standard measure; 1 EU is equal to 0.2 ng FDA reference standard Endotoxin EC-2 'RSE') per dose, and preferably <0.1 EU per dose.

Compositions of the invention are preferably gluten free.

Compositions of the invention are preferably sterile.

Compositions of the invention are preferably in aqueous form. During manufacture, dilution of the antigens to give desired final concentrations will usually be performed with WFI (water for injection).

Residual material from individual antigenic components may also be present in trace amounts in a final vaccine composition of the invention. For example, if formaldehyde is used to prepare the toxoids of diphtheria, tetanus and pertussis then the final vaccine product may retain trace amounts of formaldehyde (*e.g.* less than 10µg/ml, preferably <5µg/ml). Media or stabilizers may have been used during poliovirus preparation (*e.g*. Medium 199), and these may carry through to the final vaccine. Similarly, free amino acids (*e.g*. alanine, arginine, aspartate, cysteine and/or cystine, glutamate, glutamine, glycine, histidine, proline and/or hydroxyproline, isoleucine, leucine, lysine, methionine, phenylalanine, serine, threonine, tryptophan, tyrosine and/or valine), vitamins (*e.g.* choline, ascorbate, *etc*.), disodium phosphate, monopotassium phosphate, calcium, glucose, adenine sulfate, phenol red, sodium acetate, potassium chloride, *etc.* may be retained in the final vaccine at ≤100µg/ml, preferably <10µg/ml, each. Other components from antigen preparations, such as neomycin (*e.g*. neomycin sulfate, particularly from the IPV component), polymyxin B (*e.g*. polymyxin B sulfate, particularly from the IPV component), *etc.* may also be present at sub-nanogram amounts per dose. A further possible component of the final vaccine which originates in the antigen preparations arises from less-than-total purification of antigens. Small amounts of *B. pertussis, C.diphtheriae, C.tetani* and *S.cerevisiae* proteins and/or genomic DNA may therefore be present. To minimize the amounts of these residual components, antigen preparations are preferably treated to remove them prior to the antigens being used in the process of the invention.

### Packaging compositions of the invention

The invention can provide bulk material which is suitable for packaging into individual doses, which can then be distributed for administration to patients. Concentrations mentioned above are typically concentrations in final packaged doses, and so concentrations in bulk vaccine may be higher (*e.g.* to be reduced to final concentrations by dilution).

Human intramuscular vaccines are generally administered as unit dose with an individual dosage volume of 0.5ml. Processes of the invention may thus comprise a step of extracting and packaging a 0.5ml sample of the mixture into a container. References to 0.5ml doses will be understood to include normal variance *e.g*. 0.5ml±0.05ml. For multidose situations, multiple dose amounts will be extracted and packaged together in a single container *e.g*. 5ml for a 10-dose multidose container (or 5.5ml with 10% overfill).

Processes of the invention may comprise a step of packaging the vaccine into containers for use. Suitable containers include vials and disposable syringes (preferably sterile ones).

Where a composition of the invention is packaged into vials, these are preferably made of a glass or plastic material. The vial is preferably sterilized before the composition is added to it. To avoid problems with latex-sensitive patients, vials are preferably sealed with a latex-free stopper. The vial may include a single dose of vaccine, or it may include more than one dose (a 'multidose' vial) *e.g.* 10 doses. When using a multidose vial, each dose should be withdrawn with a sterile needle and syringe under strict aseptic conditions, taking care to avoid contaminating the vial contents. Preferred vials are made of colorless glass.

A vial can have a cap (*e.g.* a Luer lock) adapted such that a pre-filled syringe can be inserted into the cap, the contents of the syringe can be expelled into the vial (*e.g.* to reconstitute lyophilised material therein), and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed.

Where the composition is packaged into a syringe, the syringe will not normally have a needle attached to it, although a separate needle may be supplied with the syringe for assembly and use. Safety needles are preferred. 1-inch 23-gauge, 1-inch 25-gauge and 5/8-inch 25-gauge needles are typical. Syringes may be provided with peel-off labels on which the lot number and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of butyl rubber. If the syringe and needle are packaged separately then the needle is preferably fitted with a butyl rubber shield. Grey butyl rubber is preferred. Useful syringes are those marketed under the trade name "Tip-Lok"™.

Where a glass container (*e.g*. a syringe or a vial) is used, then it is preferred to use a container made from a borosilicate glass rather than from a soda lime glass.

After a composition is packaged into a container, the container can then be enclosed within a box for distribution *e.g.* inside a cardboard box, and the box will be labeled with details of the vaccine *e.g.* its trade name, a list of the antigens in the vaccine (*e.g.* 'hepatitis B recombinant', *etc*.), the presentation container (*e.g.* 'Disposable Prefilled Tip-Lok Syringes' or '10 x 0.5 ml Single-Dose Vials'), its dose (*e.g.* 'each containing one 0.5ml dose'), warnings (*e.g*. 'For Adult Use Only' or 'For Pediatric Use Only'), an expiration date, an indication, a patent number, *etc.* Each box might contain more than one packaged vaccine e.g. five or ten packaged vaccines (particularly for vials). If the vaccine is contained in a syringe then the package may show a picture of the syringe.

The vaccine may be packaged together (*e.g.* in the same box) with a leaflet including details of the vaccine *e.g.* instructions for administration, details of the antigens within the vaccine, *etc.* The instructions may also contain warnings *e.g*. to keep a solution of adrenaline readily available in case of anaphylactic reaction following vaccination, *etc.*

A packaged vaccine is preferably stored at between 2°C and 8°C. It should not be frozen.

Vaccines can be provided in full-liquid form (*i.e.* where all antigenic components are in aqueous solution or suspension) during manufacture, or they can be prepared in a form where some components are in liquid form and others are in a lyophilized form. Thus a final vaccine can be prepared extemporaneously at the time of use by mixing together two components: (a) a first component comprising aqueous antigens; and (b) a second component comprising lyophilized antigens. The two components are preferably in separate containers (*e.g*. vials and/or syringes), and the invention provides a kit comprising components (a) and (b). This format is particularly useful for vaccines that include a conjugate component, particularly Hib and/or meningococcal and/or pneumococcal conjugates, as these may be more stable in lyophilized form (whereas D, T, P and HBsAg components are preferably in liquid form). Thus conjugates may be lyophilised prior to their use with the invention. Further components may also be added prior to freeze-drying *e.g*. as stabilizers. Preferred stabilizers for inclusion are lactose, sucrose and mannitol, as well as mixtures thereof *e.g.* lactose/sucrose mixtures, sucrose/mannitol mixtures, *etc.* The final vaccine may thus contain lactose and/or sucrose. Using a sucrose/mannitol mixture can speed up the drying process.

Thus the invention provides a process for preparing a two-container combination vaccine, comprising the following steps:
- preparing an aqueous combination vaccine as described above, but wherein the said one or more antigens does not include a conjugated capsular saccharide antigen;
- packaging said aqueous combination vaccine in a first container (*e.g*. a syringe);
- preparing a conjugated capsular saccharide antigen in lyophilised form;
- packaging said lyophilised antigen in a second container (*e.g*. a vial); and
- packaging the first container and second container together in a kit.

The kit can then be distributed to physicians. In the physician's office prior to vaccination, the contents of the two containers will be mixed to yield a single liquid vaccine that is ready for administration.

### Methods of treatment and administration of the vaccine

Compositions of the invention are suitable for administration to human patients, and the invention provides a method of raising an immune response in a patient, comprising the step of administering a composition of the invention to the patient. Compositions of the invention are preferably administered to patients in 0.5ml doses (as discussed above).

The invention also provides a composition of the invention for use in medicine. The invention also provides the use of the composition of the invention in the prevention of at least whooping cough.

The invention also provides the use of antigenic components as described herein (including acellular pertussis antigens of the invention) in the manufacture of a vaccine.

In order to have full efficacy, a typical primary immunization schedule for a child may involve administering more than one dose. For example, doses may be at: 0 & 6 months (time 0 being the first dose); at 0, 1, 2 & 6 months; at day 0, day 21 and then a third dose between 6 & 12 months; at 2, 4 & 6 months; at 3, 4 & 5 months; at 6, 10 & 14 weeks; or at 0, 1, 2, 6 & 12 months. Paediatric compositions can also be used as booster doses *e.g.* for children, in the second year of life.

Adolescent booster vaccine compositions of the invention are administered in a single dose to persons of age 10 and older.

Compositions of the invention can be administered by intramuscular injection e.g. into the arm or leg.

Vaccines produced by the invention may be administered to patients at the same time as a separate vaccine *e.g.* at the same time as a pneumococcal conjugate vaccine such as PREVNAR™, at the same time as an influenza vaccine, at the same time as a rotavirus vaccine, at the same time as a MMR vaccine, *etc.*

Where compositions of the invention include an aluminium-based adjuvant, settling of components may occur during storage. The composition should therefore be shaken prior to administration to a patient. The shaken composition will be a turbid white suspension.

### General

The term "comprising" encompasses "including" as well as "consisting" *e.g*. a composition "comprising" X may consist exclusively of X or may include something additional *e.g*. X + Y.

The word "substantially" does not exclude "completely" *e.g*. a composition which is "substantially free" from Y may be completely free from Y. Where necessary, the word "substantially" may be omitted from the definition of the invention.

The term "about" in relation to a numerical value *x* is optional and means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where an antigen is described as being "adsorbed" to an adjuvant, it is preferred that at least 50% (by weight) of that antigen is adsorbed *e.g.* 50%, 60%, 70%, 80%, 90%, 95%, 98% or more. It is preferred that diphtheria toxoid and HBsAg are both at least 90% adsorbed, and ideally are totally adsorbed *i.e.* none is detectable in supernatant after cetrifugation.

### DESCRIPTION OF THE DRAWINGS

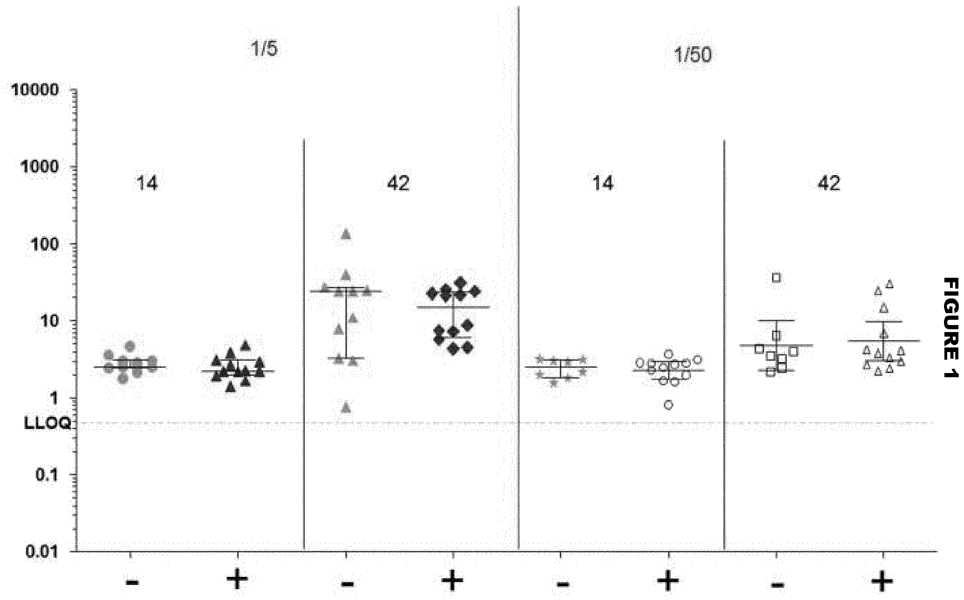

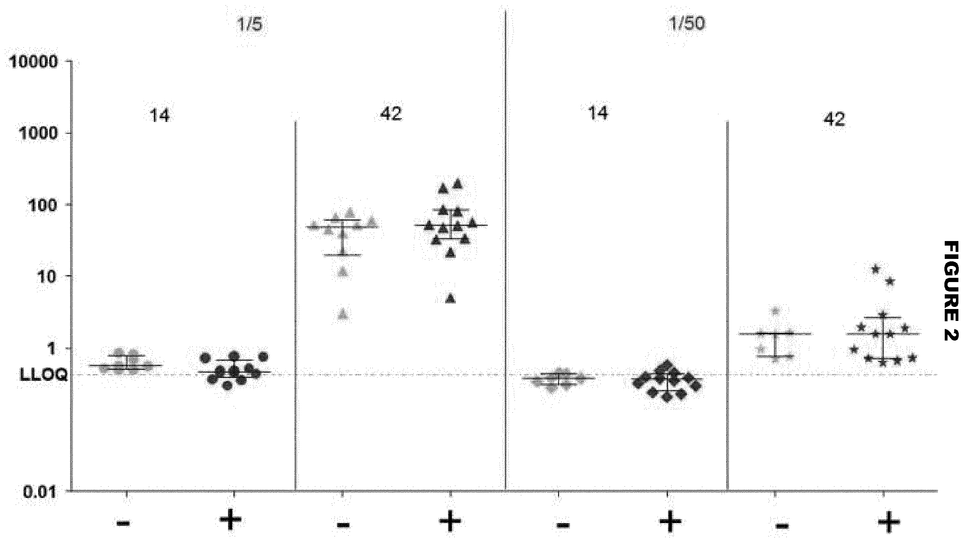

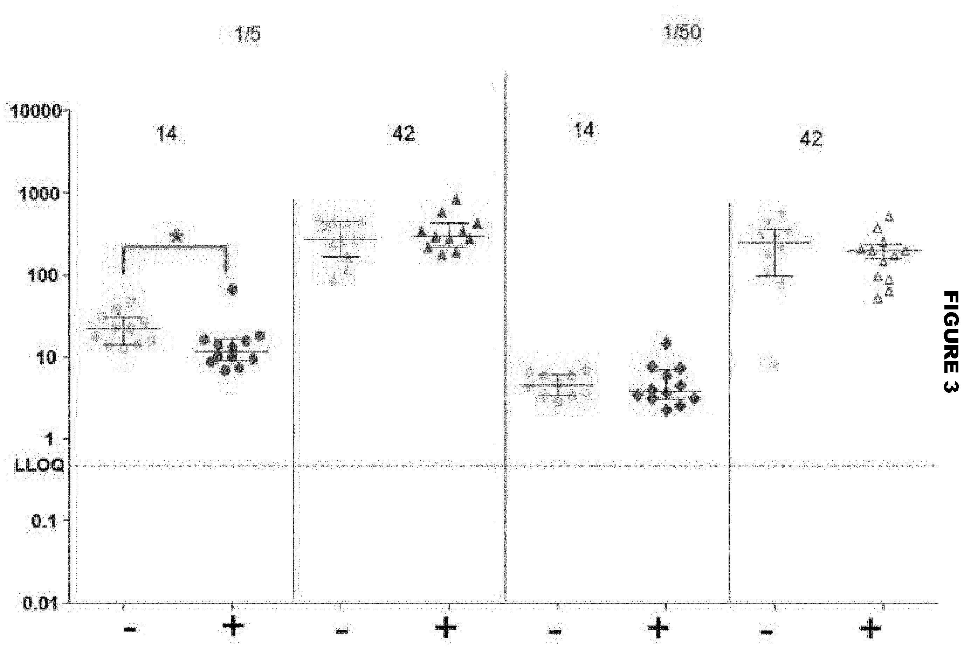

Figures 1 to 3 show IgG titres in mice immunised with a Tdap formulation containing either non-formylated or formylated aP antigens, as described in Example 6 below. IgG tires were determined by Luminex assay. Results are expressed in relative light units (RLU)/ml. The lower limit of quantification (LLOQ) is shown as a dotted line. Titres are shown for pertactin (Fig. 1), FHA (Fig. 2), and PT (Fig. 3). The numbers 1/5 or 1/50 indicate the antigen dose, relative to a human dose. The numbers 14 and 42 indicate the day after first immunisation on which the serum were assessed. The symbol + indicates a formylated antigen, whereas a - indicates a non-formylated antigen. A * indicates p = 0.02.

### CLAUSES

Clause 1. A combination vaccine comprising a genetically detoxified pertussis toxin and one or more additional antigen selected from IPV, HBsAg and Hib, characterised in that the genetically detoxified pertussis toxin is not treated with formaldehyde or glutaraldehyde.
Clause 2. The combination vaccine of clause 1, wherein the pertussis toxin is not treated with an aldehyde cross-linking agent.
Clause 3. The combination vaccine of clause 1, wherein the pertussis toxin is not treated with a cross-linking agent.
Clause 4. A combination vaccine comprising *B. pertussis* antigens PT, FHA and pertactin, characterised in that at least two of the *B. pertussis* antigens are not treated with formaldehyde or glutaraldehyde, with the proviso that, if PT is not treated with a cross-linking agent, PT is a genetically detoxified pertussis toxin.
Clause 5. The combination vaccine of clause 4, wherein the at least two of the *B.pertussis* antigens are not treated with an aldehyde cross-linking agent.
Clause 6. The combination vaccine of clause 4, wherein the at least two of the *B.pertussis* antigens are not treated with a cross-linking agent.
Clause 7. A combination vaccine comprising an aluminium salt adjuvant and at least two non-cross-linked *B. pertussis* antigens selected from PT, FHA and pertactin with the proviso that any PT is genetically detoxified.
Clause 8. The combination vaccine of clause 7, wherein the concentration of Al⁺⁺⁺ is less than 1 mg/ml.
Clause 9. The combination vaccine of clause 7, wherein the vaccine further comprises a TLR4 agonist.
Clause 10. The combination vaccine of clause 9, wherein the vaccine includes AS04 as adjuvant.
Clause 11. A combination vaccine comprising at least two non-cross-linked *B. pertussis* antigens selected from PT, FHA and pertactin and an oil-in-water emulsion adjuvant with the proviso that any PT is genetically detoxified.
Clause 12. The combination vaccine of clause 11, wherein the oil-in-water emulsion adjuvant includes MF59 and/or AS03.
Clause 13. A preservative-free combination vaccine comprising at least two non-cross-linked *B. pertussis* antigens selected from PT, FHA and pertactin with the proviso that any non-cross-linked PT is genetically detoxified.
Clause 14. A combination vaccine comprising at least two non-cross-linked *B. pertussis* antigens selected from PT, FHA and pertactin with the proviso that any PT is genetically detoxified, wherein the weight ratio of PT:FHA:pertactin is 1:1:2 or 16:16:5 or 5:10:6 or 20:20:3 or 25:25:8 or 10:5:3.
Clause 15. A combination vaccine comprising the following components:
   - D, T, aP, IPV
   - D, T, aP, HBsAg
   - D, T, aP, Hib
   - D, T, aP, Hib, IPV
   - D, T, aP, HBsAg, Hib
   - D, T, aP, HBsAg, IPV
   - D, T, aP, HBsAg, IPV, Hib
   - D, T, aP, HBsAg, IPV, Hib, Spn
   - D, T, aP, HBsAg, IPV, Hib, MenC
   - D, T, aP, HBsAg, IPV, Hib, MenC, MenA
   - D, T, aP, HBsAg, IPV, Hib, MenC, MenY
   - D, T, aP, HBsAg, IPV, Hib, MenC, MenW135
   - D, T, aP, HBsAg, IPV, Hib, MenC, MenA, MenW135, MenY
   wherein the aP component comprises at least two non-cross-linked *B. pertussis* antigens selected from PT, FHA and pertactin with the proviso that any PT is genetically detoxified.
Clause 16. A combination vaccine comprising D, T, aP, wherein the ratio of D to T measured in Lf units is between 2:1 and 3:1 and the aP component comprises at least two non-cross-linked *B. pertussis* antigens selected from PT, FHA and pertactin with the proviso that any PT is genetically detoxified.
Clause 17. A combination vaccine comprising D, T, aP, wherein the ratio of T to D measured in Lf units is greater than 1.5 and the aP component comprises at least two non-cross-linked *B. pertussis* antigens selected from PT, FHA and pertactin with the proviso that any PT is genetically detoxified.
Clause 18. The combination vaccine of any one of clauses 4-17, wherein one of the *Bordetella pertussis* antigens is a genetically detoxified pertussis toxin.
Clause 19. The combination vaccine of any one of clauses 1-18, wherein the genetically detoxified pertussis toxin is PT-9K/129G.
Clause 20. A process for preparing an aP component comprising:
   a. growing a culture of a *B*. *pertussis* strain expressing a genetically detoxified pertussis toxin;
   b. purifying two or more *B. pertussis* antigens from the culture to obtain two or more batches each containing a different purified *B. pertussis* antigen; and
   c. mixing the two or more batches to prepare the aP component;
   wherein the process is characterised in that the purified *B. pertussis* antigens are not treated with a cross-linking agent.
Clause 21. The process of clause 20, wherein the genetically detoxified pertussis toxin is PT-9K/129G.
Clause 22. A process for preparing an aP component comprising:
   d. growing a culture of a *B. pertussis* strain in which the gene encoding pertussis toxin has been deleted;
   e. purifying two or more *B. pertussis* antigens from the culture to obtain two or more batches each containing a different purified *B. pertussis* antigen; and
   f. mixing the two or more batches to prepare the aP component;
   wherein the process is characterised in that the purified *B. pertussis* antigens are not treated with a cross-linking agent.
Clause 23. A process for preparing an aP component comprising:
   g. growing a culture of a *B. pertussis* strain;
   h. purifying two or more *B. pertussis* antigens from the culture to obtain two or more batches each containing a different purified *B. pertussis* antigen; and
   i. mixing the two or more batches to prepare the aP component;
   wherein the process is characterised in that only the batch containing purified enzymatically active PT is treated with a cross-linking agent, but the batches containing other purified *B. pertussis* antigens are not treated with a cross-linking agent.
Clause 24. A process for manufacturing a combination vaccine comprising mixing the aP component obtained by any one of clauses 19-23 with one or more non-pertussis antigen(s) to prepare a combination vaccine.
Clause 25. A combination vaccine obtainable by the process of clause 24.

### MODES FOR CARRYING OUT THE INVENTION

### Example 1: Stability of formylated and non-formylated FHA batches

Two FHA batches were prepared by purifying FHA from the supernatant of a *B. pertussis* culture. The supernatant was concentrated and diafiltrated. The filtered concentrate was added onto a hydroxyapatite column. The FHA-containing eluate was further purified by a series of chromatographic steps including a butyl 650M Sepharose column and a Q Sepharose FF column. The resulting purified FHA batches were concentrated and subjected to diafiltration. One FHA batch was additionally incubated in the presence of formaldehyde and lysine, the other batch was left untreated.

Samples of each batch were incubated at room temperature (RT) for up to one month or at 2-8°C for up to three months. The initial protein concentration of each sample was determined by measuring the adsorption at 280 nm. The measurements were repeated after incubation for two weeks and one month (samples incubated at RT) or two weeks, one month and three months (samples incubated at 2-8°C). Structural stability was confirmed by 3-8% gradient SDS-PAGE. Stability at RT and 2-8°C was comparable for both the formaldehyde-treated batch and the untreated batch. No statistically significant reduction of the protein concentration was observed for both batches after one month at RT and one or three months at 2-8°C.

Integrity of the samples was also confirmed by size exclusion high pressure liquid chromatography (SEC-HPLC) using a Superdex 200® HPLC column. Samples were filtered before running them over the column. Again no difference was apparent between the formaldehyde-treated and the untreated batch after incubating samples of each batch for one or three months at 2-8°C.

### Example 2: Aggregate formation in formylated and non-formylated FHA batches

Formation of FHA aggregates/precipitates during incubation was checked by dynamic light scattering (DLS). Samples were centrifuged before testing. Two peaks were identified: Peak 1 corresponded to the monomeric form and peak 2 corresponded to the aggregate form. A 60°C angle was used to evaluate aggregate/precipitate formation. DLS analysis revealed that precipitation occurred during storage at 2-8°C for 3 months whether the FHA was treated with formaldehyde or not. Precipitation could also be reduced by the addition of 0.05% Tween-80 to the formaldehyde-treated sample but not the untreated FHA sample. Aggregation only occurred subsequent to formaldehyde treatment, and further studies showed that aggregation could be reduced by lowering the concentration of formaldehyde used to treat FHA.

### Example 3: Stability of vaccine composition containing formylated or non-formylated FHA

Two TdaP vaccine compositions were formulated comprising the following antigens: Tetanus Toxoid (T), Diphtheria toxoid (D), and three purified antigens from acellular Pertussis (aP) (PT, FHA and pertactin). Vaccines were formulated in histidine buffer (100 mM, pH 6.5) supplemented with NaCl (9 mg/ml) and adjuvanted with aluminium hydroxide (2 mg/ml). The FHA in one of the vaccine compositions was derived from a formaldehyde-treated batch, while the FHA in the other vaccine composition was FHA from a batch that had been left untreated.

Short-term stability studies were performed by incubating samples from each of the two vaccine compositions containing formylated and non-formylated FHA, respectively, for 2 or 4 weeks at 2-8°C, and at 36-38°C).

Degree of adsorption of the aP antigens was quantitatively determined using sandwich ELISA. The results are summarised in Tables 1 and 2. No difference in the degree of adsorption was observed between TdaP vaccine compositions formulated with non-formylated or formylated FHA bulks at time 0 and after up to one month incubation at either 2-8°C or 36-38°C. Integrity/identity of the samples after incubation was confirmed by Western blotting using PT-, FHA-, and pertactin-specific antibodies. No differences were observed between the samples at time 0 and after two weeks or one month incubation at 2-8°C and 36-38 °C, respectively, whether the vaccine composition contained formylated or non-formylated FHA.

**Table 1: Stability results for Tdap vaccine containing non-formylated FHA:**

| **Analytical methods** | **Acceptable range** | **Time 0** | **2 weeks at 36-38°C** | **4 weeks at 36-38°C** | **2 weeks at 2-8°C** | **4 weeks at 2-8°C** |
|---|---|---|---|---|---|---|
| **pH** | 6.0 - 7.0 | 6.4 | 6.5 | 6.5 | 6.5 | 6.4 |
| **Degree of adsorption for DT** | N/A | > 90 % | > 90 % | > 90 % | > 90 % | > 90 % |
| **Degree of adsorption for TT** | N/A | > 90 % | > 90 % | > 90 % | > 90 % | > 90 % |
| **Degree of adsorption for PT (ELISA Test)** | N/A | > 99.84% | > 99% | > 99% | > 99% | > 99% |
| **Degree of adsorption for FHA (ELISA Test)** | N/A | >99.84% | > 99% | > 99% | > 99% | > 99% |
| **Degree of adsorption for 69K (ELISA Test)** | N/A | >99.84% | > 99% | > 99% | > 99% | > 99% |

**Table 2: Stability results for Tdap vaccine containing formylated FHA:**

| **Analytical methods** | **Acceptable range** | **Time 0** | **2 weeks at 36-38°C** | **4 weeks at 36-38°C** | **2 weeks at 2-8°C** | **4 weeks at 2-8°C** |
|---|---|---|---|---|---|---|
| **pH** | 6.0 - 7.0 | 6.4 | 6.5 | 6.5 | 6.5 | 6.4 |
| **Degree of adsorption for DT** | N/A | >90% | >90% | >90% | >90% | >90% |
| **Degree of adsorption for TT** | N/A | >90% | >90% | >90% | >90% | >90% |
| **Degree of adsorption for PT (ELISA Test)** | N/A | > 99% | > 99% | > 99% | > 99% | > 99% |
| **Degree of adsorption for FHA (ELISA Test)** | N/A | > 99% | > 99% | > 99% | > 99% | > 99% |
| **Degree of adsorption for 69K (ELISA Test)** | N/A | > 99% | > 99% | > 99% | > 99% | > 99% |

### Example 4: In vivo immunogenicity properties (ELISA) of aP antigens

Groups of eight CD1 mice were immunized subcutaneously with TdaP vaccine compositions comprising either non-formylated or formylated FHA (dosage 1). In addition, the tested TdaP vaccines were diluted 4-fold (dosage 2) and 16-fold (dosage 3) in saline, and the dilutions were used immediately after preparation to inject the additional mice subcutaneously. All of the tested vaccines were prepared using the same batch of formylated PT and formylated pertactin.

The mice were bled five weeks following the injection. Sera from the bleeds were used to perform ELISA assays. In order to assess the efficacy of the two TdaP vaccines containing formylated or non-formylated FHA, respectively, the immunogenicity of each of the aP antigens was determined by measuring the mean geometric antibody titre against PT, FHA and pertactin. The results of the ELISA assays are summarised in Tables 3 to 5.

**Table 3: Immunogenicity results (GMT (UI/ml) for PT antigen:**

| **FHA treatment** | **Dosage 1** | **Dosage 2** | **Dosage 3** |
|---|---|---|---|
| **Non-formylated** | 220 | 218 | 51 |
| **Formylated** | 194 | 120 | 36 |

**Table 4: Immunogenicity results (GMT (UI/ml) for FHA antigen:**

| **FHA treatment** | **Dosage 1** | **Dosage** 2 | **Dosage** 3 |
|---|---|---|---|
| **Non-formylated** | 124 | 53 | 5 |
| **Formylated** | 167 | 71 | 5 |

**Table 5: Immunogenicity results (GMT (UI/ml) for pertactin antigen:**

| **FHA treatment** | **Dosage 1** | **Dosage 2** | **Dosage 3** |
|---|---|---|---|
| **Non-formylated** | 52 | 91 | 5 |
| **Formylated** | 97 | 52 | 3 |

Immunogenicity results of Tdap vaccine compositions containing non-formylated and formylated FHA were very similar, suggesting that omission of the formaldehyde-treatment step has no effect on the immunogenicity of the FHA component itself or on the PT and pertactin components.

### Example 5: Relative potency of aP antigens

Single mouse titres (IU/ml) were used to determine the relative potency of each vaccine formulation compared to a reference Tdap vaccine using the methodology described in the European Pharmacopoeia. Results are summarised in Table 6.

**Table 6: Relative potency results (Potency (95%CI)):**

| **FHA treatment** | **PT** | **FHA** | **Pertactin** |
|---|---|---|---|
| **Non-formylated** | 0.98 (0.51-1.88) | 0.40 (0.21-0.69) | 0.78 (0.38-1.55) |
| **Formylated** | 0.55 (0.31-0.92) | 0.49 (0.30-0.76) | 0.74 (0.45-1.21) |

The omission of the formaldehyde-treatment step had no effect on the relative potency of the FHA and pertactin components in the tested Tdap vaccine. The unexpected higher PT potency in vaccine comprising non-formylated FHA will require further investigation.

### Example 6: In vivo immunogenicity properties of non-formulated and formylated aP antigens

To confirm that the above results obtained for non-formylated FHA were similarly applicable to other antigens commonly included in the aP antigen component of Tdap vaccines, two batches of an experimental Tdap vaccine were prepared, in which the three aP antigens were either formylated or non-formylated.

Five groups of 12 mice each (female, Balb/C mice, 6 weeks old) were immunised as follows: Groups 1 and 2 received a Tdap vaccine in the three aP antigens were non-formylated, whereas groups 3 and 4 received a Tdap vaccine in which all of the aP antigens were formylated. Mice in groups 1 and 3 received 1/5 of the human dose of the Tdap vaccine, but mice in groups 2 and 4 received 1/50 of the human dose. Mice were injected i.m. twice (days 0 and 28) with 100µl (2 × 50µl each time). Mice in group 5 were not immunised and served as a naive control group.

Pre-immunisation serum samples were taken on day 0. Post-immunisation serum samples were taken on days 14 and 42, and serum IgG titres were determined for the tested aP antigen (see Figures 1-3). Titres were statistically assessed by test t, Mann-Whitney test.

With the exception of PT at day 14, no statistically significant differences in IgG titres were observed between batches containing only formylated aP antigens and batches that contained one non-formylated aP antigen. For PT, the post-immunisation IgG titres at 1/5 of the human dose at day 14 were statistically significantly higher for the non-formylated antigen than the IgG titres obtained with the formylated antigen (see Figure 3).

As expected, for each of the investigated antigens post-immunisation IgG titres at day 42 were statistically significantly higher than post-immunisation IgG titres at day 14. Similarly, titres at day 42 were in all cases significantly higher than in the naive mice, even at 1/50 dose (p ≤ 0.003 in all cases), and titres at 1/5 dose were significantly higher than with a 1/50 dose (p ≤ 0.01 in all cases).

It will be understood that the invention has been described by way of example only and modifications may be made whilst remaining within the scope and spirit of the invention.

### REFERENCES

*[1]* Vaccines. (eds. Plotkin & Orenstein). 4th edition, 2004, ISBN: 0-7216-9688-0
[2] Rappuoli et al. (1991) TIBTECH 9:232-238
[3] Nencioni et al. (1990) Infect Immun 58(5):1308-15
[4] Nencioni et al. (1991) Infect Immun 59(2):625-30
[5] Burnette et al. (1988) Science 242:72-74
[6] Loosmore et al. (1990) Infect Immun 58:3653-366
[7] Cockle (1989) FEBS Letters 249:329-332
[8] US patent 7,427,404
[9] Edwards et al. (1995) Pediatrics 96(3 Pt 2):548-57
[10] Pichichero et al. (1997) Pediatrics 100(5):772-88
[11] Di Tommaso et al. (1994) InfectImmun 62(5):1830-4
[12] Denoël et al. (2002) Vaccine 20:2551-5.
[13] WO1994/03206
[14] Module 1 of WHO's *The immunological basis for immunization series* (Galazka)
[15] Lyng (1990) Biologicals 18:11-17
[16] NIBSC code: 69/017
[17] NIBSC code: DIFT
[18] Sesardic et al. (2001) Biologicals 29:107-22
[19] NIBSC code: 98/560
[20] Kuhmlann & Rieger (1995) Immunol Infect Dis 5:10-4
[21] NIBSC code: TEFT
[22] Sesardic et al. (2002) Biologicals 30:49-68
[23] NIBSC code: 98/552
[24] Ramsay et al. (2001) Lancet 357(9251):195-196
[25] Lindberg (1999) Vaccine 17 Suppl 2:S28-36
[26] Buttery & Moxon (2000) JR Coll Physicians Lond 34:163-168
[27] Ahmad & Chapnick (1999) Infect Dis Clin North Am 13:113-133, vii
[28] Goldblatt (1998) J. Med. Microbiol. 47:563-567
[29] EP-A-0477508
[30] US patent 5,306,492
[31] WO1998/42721
*[32]* Conjugate Vaccines (eds. Cruse et al.) ISBN 3805549326, particularly vol. 10:48-114
[33] Hermanson (1996) Bioconjugate Techniques ISBN: 0123423368 or 012342335X
[34] Lees et al. (1996) Vaccine 14:190-198
[35] WO1995/08348
[36] WO1998/42721
[37] US patent 4,882,317
[38] US patent 4,695,624
[39] EP-B-0477508
[40] Mol. Immunol. 1985, 22, 907-919
[41] EP-A-0208375
[42] WO2000/10599
[43] Gever et al. (1979) Med. Microbiol. Immunol. 165:171-288
[44] US patent 4,057,685
[45] US patent 4,673,574
[46] US patent 4,761,283;
[47] US patent 4,808,700
[48] US patent 4,459,286
[49] US patent 4,965,338
[50] US patent 4,663,160
[51] US patent 4,356,170
*[52]* Research Disclosure, 453077 (Jan 2002)
[53] Anderson (1983) Infect Immun 39(1):233-238
[54] Anderson et al. (1985) J Clin Invest 76(1):52-59
[55] Kanra et al. (1999) The Turkish Journal of Paediatrics 42:421-427
[56] Ravenscroft et al. (2000) Dev Biol (Basel) 103: 35-47
[57] WO96/40242
[58] Giuliani et al. (2006) PNAS USA 103:10834-9
[59] W.H.O. Tech. Rep. Ser. 594:51, 1976
[60] Zielen et al. (2000) Infect. Immun. 68:1435-1440
[61] Darkes & Plosker (2002) Paediatr Drugs 4:609-630
[62] Watson (2000) Pediatr Infect Dis J 19:331-332
[63] Rubin (2000) Pediatr Clin North Am 47:269-285, v
[64] Jedrzejas (2001) Microbiol Mol Biol Rev 65:187-207
[65] Vanlandschoot et al. (2005) J Gen Virol 86:323-31
[66] WO2007/054820
[67] WO2003/066094
[68] Module 6 of WHO's *The immunological basis for immunization series* (Robertson)
[69] WO2008/028956
[70] WO2008/028957
[71] Liao et al. (2012) J Infect Dis. 205:237-43
[72] EP-A-0372501
[73] EP-A-0378881
[74] EP-A-0427347
[75] WO1993/17712
[76] WO1994/03208
[77] WO1998/58668
[78] EP-A-0471177
[79] WO1991/01146
[80] Falugi et al. (2001) Eur J Immunol 31:3816-24
[81] Baraldo et al. (2004) Infect Immun 72:4884-87
[82] EP-A-0594610
[83] WO2000/56360
[84] WO2002/091998
[85] Kuo et al. (1995) Infect Immun 63:2706-13
[86] WO2001/72337
[87] WO2000/61761
[88] WO2004/041157
[89] Vaccine Design: The Subunit and Adjuvant Approach (eds. Powell & Newman) Plenum Press 1995 (ISBN 0-306-44867-X)
[90] WO1997/00697
[91] WO2002/00249
[92] US patent 6,013,264
[93] US patent 4,624,918
[94] Vaccine Adjuvants: Preparation Methods and Research Protocols (Volume 42 of Methods in Molecular Medicine series). ISBN: 1-59259-083-7. Ed. O'Hagan.
[95] WO2011/067669.
[96] WO2011/067672.
[97] WO2011/067673.
[98] WO2008/056263.
[99] WO2011/154442.
[100] WO2011/154443.
[101] WO2011/154444.
[102] WO90/14837.
[103] Podda & Del Giudice (2003) Expert Rev Vaccines 2:197-203.
[104] Podda (2001) Vaccine 19: 2673-2680.
[105] WO2008/043774.
[106] WO2005/097181.
[107] WO95/11700.
[108] US-2007/014805.
[109] WO2007/080308.
[110] WO2010/023551
[111] Brito et al. (2011) Vaccine 29:6262-6268.
[112] US-8092813.
[113] WO2011/141819.
[114] US-6630161.
[115] Rosenberg et al. (2010) J Immunol 184:136.20
[116] US patent 4,666,886
[117] WO2009/118296
[118] WO2008/005555
[119] WO2009/111337
[120] WO2009/067081
[121] WO2007/040840
[122] WO2010/014913
[123] WO2012/031140.
[124] Steinhagen et al. (2011) Vaccine 29:3341-55.
[125] GB-A-2220211.
[126] Myers et al. (1990) pages 145-156 of Cellular and molecular aspects of endotoxin reactions*.*
[127] Ulrich (2000) Chapter 16 (pages 273-282) of reference 94.
[128] Johnson et al. (1999) J Med Chem 42:4640-9.
[129] Baldrick et al. (2002) Regulatory Toxicol Pharmacol 35:398-413.
[130] Johnson et al. (1999) Bioorg Med Chem Lett 9:2273-2278.
[131] Evans et al. (2003) Expert Rev Vaccines 2:219-229.
[132] Bazin et al. (2006) Tetrahedron Lett 47:2087-92.
[133] Wong et al. (2003) J Clin Pharmacol 43(7):735-42.
[134] US2005/0215517.
[135] Coler et al. (2011) PLoS ONE 6(1):e16333.
[136] WO03/011223.
[137] WO2007/053455.
[138] Garçon et al. (2007) Expert Rev Vaccines 6:723-39.
[139] WO 94/21292.
[140] WO2010/144734.
[141] Nony et al. (2001) Vaccine 27:3645-51

## Claims

1. A process for preparing an acellular pertussis (aP) component comprising:
a. growing a culture of a *B*. *pertussis* strain expressing a genetically detoxified pertussis toxin;
b. purifying two or more *B. pertussis* antigens from the culture to obtain two or more batches each containing a different purified *B. pertussis* antigen; and
c. mixing the two or more batches to prepare the aP component;
wherein the process is **characterised in that** the purified *B. pertussis* antigens are not treated with a cross-linking agent.

2. The process of claim 1, wherein the genetically detoxified pertussis toxin is PT-9K/129G.

3. A process for manufacturing a combination vaccine comprising mixing the aP component obtained by any one of claims 1-2 with one or more non-pertussis antigen(s) to prepare a combination vaccine.

4. A combination vaccine obtainable by the process of claim 4.
